# EUROPEAN PATENT SPECIFICATION

(11) **EP 1 309 707 B1**
(45) Date of publication and mention of the grant of the patent: **04.04.2007**
(21) Application number: 01955134.0
(22) Date of filing: 06.08.2001
(51) Int. Cl.: C12N 15/65

(54) **ASSAYS FOR ALTERNATIVE LENGTHENING OF TELOMERES IN HUMAN CELLS**
TESTVERFAHREN ZUR BESTIMMUNG DER ALTERNATIVEN VERLANGERUNG VON TELOMEREN IN MENSCHLICHEN ZELLEN
ANALYSES DE VARIANTES D'ALLONGEMENT DE TELOMERES DANS CELLULES HUMAINES

(30) Priority: 07.08.2000 AU PQ923400
(43) Date of publication of application: 14.05.2003
(73) Proprietor: Children's Medical Research Institute, Westmead, NSW 2145 (AU)
(72) Inventor: REDDEL, Roger, Robert, St. Ives, NSW 2075 (AU); DUNHAM, Melissa, Ann, Sorrento, W.A. 6020 (AU); FASCHING, Clare, Louise, Camperdown, NSW 2050 (AU); NEUMANN, Axel, Arthur, Chippendale, NSW 2008 (AU)
(74) Representative: Gallagher, Kirk James
(86) International application number: PCT/AU2001/000954
(87) International publication number: WO 2002/012515

(56) References cited:
- REDDEL R R ET AL: "Immortalized cells with no detectable telomerase activity: A review" BIOCHEMISTRY (MOSCOW), vol. 62, no. 11, November 1997 (1997-11), pages 1254-1262, XP009030227 ISSN: 0006-2979
- DUNHAM MELISSA A ET AL: "Telomere maintenance by recombination in human cells" NATURE GENETICS, vol. 26, no. 4, December 2000 (2000-12), pages 447-450, XP002281263 ISSN: 1061-4036
- LOUIS E.J.: 'Use of the expand tm PCR system to complete the telomeres for the yeast genome project' BIOCHEMICA vol. 3, 1995, pages 25 - 26, XP002965706
- LOUIS E.J. AND BORTS R.H.: 'A complete set of marked telomeres in saccharomyces cerevisiae for physical mapping and cloning' GENETICS vol. 139, 1995, pages 125 - 136, XP002964448
- SOHN J.H. ET AL.: 'A family of telomere-associated autonomously replicating sequences and their functions in targeted recombination in hansenula polymorpha DL-1' JOURNAL OF BACTERIOLOGY vol. 181, no. 3, 1999, pages 1005 - 1013, XP002964449
- SPRUNG C.N. ET AL.: 'Chromosome healing in mouse embryonic stem cells' PROC. NATL. ACAD. SCI. USA vol. 96, 1999, pages 6781 - 6786, XP002964450
- ROTH C.W. ET AL.: 'Chromosome and elongation by recombination in the mosquito anopheles gambiae' MOLECULAR AND CELLULAR BIOLOGY vol. 17, no. 9, 1997, pages 5176 - 5183, XP002964451

## Description

### Field of the invention

The present invention relates to constructs and their use in methods of assaying telomere alteration and uses of the constructs and methods to assay agents for effects on telomere alteration.

### Background to the invention

Telomeres are specialised structures located at the ends of eukaryotic linear chromosomes. These structures are highly conserved among widely divergent eukaryotes and consist of simple, tandem repeat sequences with a G rich strand running 5' to 8' towards the end of the chromosome. Telomeres function to stabilise chromosomes by protecting the ends from degradation and end-to-end fusion to other chromosomes.

During replication of linear chromosomes, conventional DNA polymerases require a primer for second strand synthesis. This generates discontinuous Okazaki fragments. The RNA primers are degraded, internal gaps are filled in, however a gap at the 5' end of the replicated strand cannot be filled resulting in incomplete replication and gradual loss of telomere length during subsequent rounds of replication. According to the telomere hypothesis of senescence, this gradual loss of telomeric sequences from the ends of chromosomes has been proposed to function as the cells' mitotic clock. However, there may be more than one clock mechanism.

Unicellular organisms and germ line cells have overcome the problem of telomere shortening by a specialised reverse transcriptase known as telomerase. It was first identified in *Tetrahymena thermophila.* Telomerase is a ribonucleoprotein complex that has an RNA motif complementary to the telomeric DNA providing a template that adds repeats to the 3' end of the G rich strand. Telomerase is present in human germ line cells, whereas very little or no telomerase is detected in most somatic cells.

However, in most normal somatic cells, the DNA at the ends of chromosomes (telomeres) shortens every time call division occurs. This appears to limit the number of times a cell can divide, and to act as a major barrier to cells becoming cancerous. Cancer cells overcome this barrier by switching on a telomere maintenance mechanism (reviewed in Colgin and Reddel, 1989). In most cases this mechanism involves the enzyme, telomerase, that synthesises new telomeric DNA to replace that which is lost and most immortalised human cell lines and tumours examined to date have telomerase activity. However, a number of immortalised cell lines and tumours have no detectable telomerase activity. Analysis of the Telomere Restriction Fragment (TRF) lengths of telomerase-negative immortalised cell lines usually shows very heterogenous lengths varying from very short to very long. Maintenance of telomere length in the absence of telomerase activity is referred to as Alternative Lengthening of Telomeres (ALT). All immortalised cell lines examined to date maintain their telomeres either by telomerase or an ALT mechanism.

There is evidence that ALT cells use (partial) recombination and copy switching to maintain their telomeres. Yeast cells defective for telomerase genes use recombination. *Saccharomyces cerevisiae* and *Kluyveromyces lactis* cells lacking the telomerase RNA gene undergo a period of senescence with survivors maintaining telomeres by a RAD-52 dependent recombination process. In this situation, some of the telomeres are longer than wild type telomeres.

The features of human cells utilising an ALT mechanism include:
(a) absence of telomerase activity (Bryan et al., 1995);
(b) very short and abnormally long telomeres present in the same cell (Bryan et al., 1995);
(c) nuclear structures containing PML protein, telomeric DNA, telomere binding proteins, and other proteins (Yeager et al., 1999).

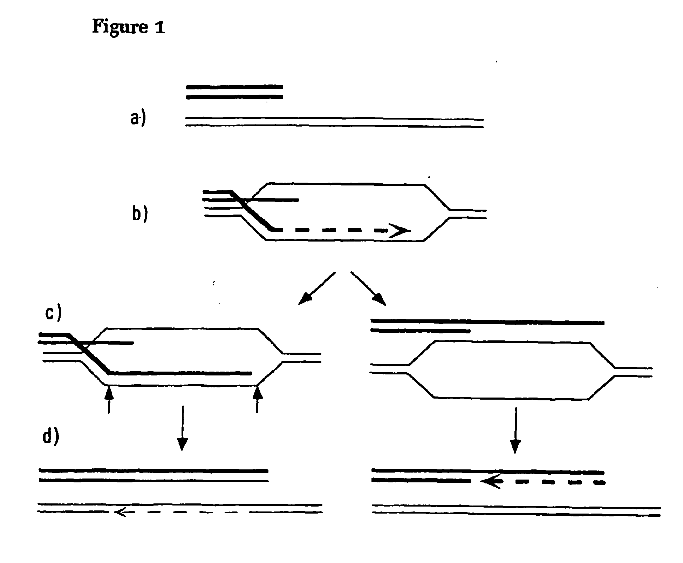

All telomeres within a cell contain essentially the same DNA sequence (in vertebrates, many hundreds of copies of a TTAGGG hexanucleotide repeat). The present inventors and others have therefore proposed (Reddel et al., 1997; Reddel, 2000) that in ALT cells, one telomere may act as the template for synthesis of telomeric sequence in another telomere (Figure 1), and, by a looping back process, in itself.

Most cancer cell use telomerase, and there is widespread interest in the development of telomerase inhibitors as a novel form of cancer treatment. The existence of an ALT mechanism, however, means (a) telomerase inhibitors are unlikely to be effective treatment for cancers that are already independent of telomerase, and (b) treatment with telomerase inhibitors may cause the emergence of resistant cancer cells that have switched on ALT. It is therefore very likely that effective telomere-directed cancer treatments will require both telomerase inhibitors and ALT inhibitors. In order to find ALT inhibitors, it is necessary to have an assay for ALT activity.

### Summary of the invention

One of the difficulties of studying recombination at the telomeres is that the DNA sequences are identical. To overcome this problem, the present inventors have taken the approach of using a plasmid with a tagged telomere sequence. This novel approach differs to other reports which used a plasmid to seed new telomeres onto truncated chromosomes caused by the integration of the plasmid. By contrast the present invention provides plasmid constructs which can be targeted to any telomere without causing a truncation to the chromosome.

Accordingly, in a general aspect, the present invention relates to genetic constructs for use in assays for alternative lengthening of telomeres (ALT) in cells.

In a first aspect, the present invention provides a nucleic acid construct capable of integrating a first DNA tag sequence into a telomere by homologous recombination, the construct comprising the DNA tag sequence linked to (i) a first DNA sequence positioned 3' of the tag sequence and (ii) a second DNA sequence positioned 5' of the tag sequence, the said first and second DNA sequences each comprising multiple repeats homologous to eukaryotic telomere DNA, and the tag sequence comprising a first marker.

Preferably the first marker is a first eukaryotic selectable marker. Preferably the multiple repeats are (TTAGGG)n.

In a further embodiment, the tag sequence further comprises a first prokaryotic selectable marker, and optionally, a bacterial origin of replication Preferably the construct comprises a first endonuclease recognition site positioned 3' to the first prokaryotic selectable marker.

The first aspect of the invention also provides a plasmid capable of integrating into a telomere by homologous integration, the plasmid comprising:
(a) two arms of DNA sequence homologous to human telomere DNA, each arm comprising multiple repeats;
(b) a DNA tag flanked by the two arms comprising a marker designed for expression in a eukaryotic cell.

The tag is incorporated telomerically (within or at the end of a telomere) in progeny of cells having ALT activity.

Preferably, the marker is a selectable marker.

Preferably, the multiple repeats are (TTAGGG)ₙ.

Preferably, the tag further comprises a bacterial origin of replication and a bacterial selectable marker.

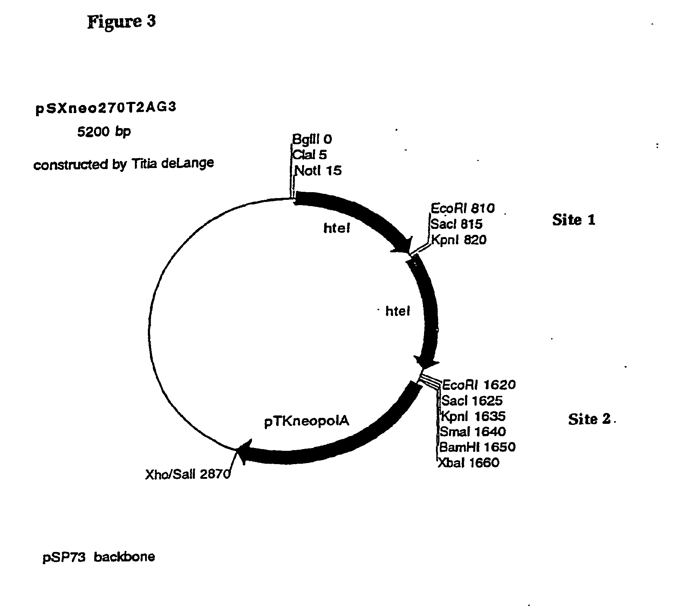

In one preferred form, the plasmid is Tel as defined in Figure 3.

In a second aspect, the present invention provides a nucleic acid construct capable of integrating a second DNA tag sequence into a subtelomeric position within a chromosome, the construct comprising the second DNA tag sequence linked to (i) a first DNA sequence positioned 8' of the second DNA tag sequence, which first DNA sequence comprises multiple repeats homologous to eukaryotic telomere DNA; and optionally (ii) a third DNA sequence positioned 5 of the tag sequence, wherein any DNA sequence 5' of the second DNA tag sequence does not contain a nucleic acid sequence homologous to eukaryotic telomere DNA, and the second DNA tag sequence comprises a second marker.

Preferably the second marker is a second eukaryotic selectable marker. Preferably the multiple repeats are (TTAGGG)n.

In a further embodiment, the second DNA tag sequence further comprises a second prokaryotic selectable marker, and optionally, a bacterial origin of replication Preferably the construct comprises a second endonuclease recognition site positioned 5' to the second prokaryotic selectable marker. It is also preferred that the multiple repeats comprise a third unique endonuclease recognition site.

The second aspect of the invention also provides a plasmid capable of integrating into a subtelomeric position, the plasmid comprising :
(a) a first arm of DNA having a nucleic acid sequence not homologous to human telomere DNA and a marker designed for expression in a eukaryotic cell ; and
(b) a second arm of DNA positioned 3' from the first arm of DNA, the second arm having a sequence homologous to human telomere DNA and comprising multiple repeats.

Preferably, the marker is a selectable marker.

Preferably, the multiple repeats are (TTAGGG)ₙ.

Preferably, the tag further comprises a bacterial origin of replication and a bacterial selectable marker.

In a third aspect, the present invention provides a host cell comprising a nucleic acid construct according to the first aspect and/or the second aspect of the invention.

Also provided is a host cell which comprises one or more first DNA tag sequences integrated into one or more telomeres; and/or one or more second DNA tag sequences integrated into one or more chromosomes at a subtelomeric position.

Preferably, the host cell has alternative lengthening of telomeres (ALT) activity.

The third aspect of the invention also provides a cell having ALT activity and containing a plasmid according to the first or second aspect of the present invention and having a telomeric tag integrated into one or more telomeres.

In a fourth aspect, the present invention provides a method of assaying for ALT in a eukaryotic cell, which method comprises:
(a) introducing into the cell a nucleic acid construct according to the first aspect of the invention comprising a first DNA tag sequence;
(b) selecting cells having the first DNA tag sequence integrated into one or more telomeres;
(c) allowing cells from (b) to undergo division; and
(d) determining the presence of the first DNA tag sequence in additional telomeres.

Preferably the DNA tag sequence comprises a first eukaryotic selectable marker and step (b) comprises incubating the cell under conditions that confer a selective growth advantage on cells that comprise the first selectable marker.

In one embodiment, step (a) further comprises introducing into the cell a nucleic acid construct according to the second aspect of the invention comprising a second DNA tag sequence, step (b) further comprises selecting cells having the second DNA tag sequence integrated into one or more telomeres; and step (d) further comprises detecting the frequency with which the first DNA tag sequence gets copied into the telomere of a chromosome that contains the second DNA tag sequence.

Preferably the second DNA tag sequence comprises a second eukaryotic selectable marker and step (b) comprises incubating the cell under conditions that confer a selective growth advantage on cells that comprise the second selectable marker.

The fourth aspect of the invention also provides an in vitro assay for ALT in a eukaryotic cell, the assay comprising:
(a) treating a target cell with a plasmid according to the first aspect of the present invention;
(b) selecting treated cells having the plasmid integrated into one or more telomeres;
(c) allowing cells from (b) to undergo division; and
(d) detecting for the incorporation of the tag from the plasmid into additional telomeres.

The selecting step (b) can be achieved by detecting the presence of a selectable marker.

The detecting step (d) can be by fluorescence *in situ* hybridisation (FISH) using a probe for the tag DNA. It will be appreciated, however, that other detection methods would also be suitable.

In a fifth aspect, the present invention provides an assay method for screening a compound for an effect on ALT activity in a cell, the method comprising:
(a) providing a cell having ALT activity and comprising a first DNA tag sequence comprising a marker, the DNA tag sequence being integrated into one or more telomeres of said cell;
(b) contacting the cell with a test compound;
(c) allowing the cell to undergo division; and
(d) determining in the progeny of the cell whether there is any change in the rate or incidence of telomeric incorporation of the first DNA tag sequence in additional telomeres as compared with untreated cells.

The fifth aspect of the invention also provides an assay for screening a compound for an effect on ALT activity in a cell, the assay comprising:
(a) providing a cell according to the third aspect of the present invention and having ALT activity;
(b) treating the cell with a test compound;
(c) allowing the cell to undergo division; and
(d) detecting for any change in the rate or incidence of telomeric incorporation of the tag from the plasmid in additional telomeres in the progeny of the cell as compared with untreated cell progeny.

A decrease in the rate or incidence of incorporation of the telomeric tag is indicative of inhibition of ALT activity by the compound. An increase in the rate or incidence of incorporation of the tag is indicative of stimulation of ALT activity by the compound. No change in the rate or incidence of incorporation of the telomeric tag is indicative of no effect on ALT activity by the compound.

In a sixth aspect, the invention provides an assay method for screening a compound for an effect on ALT activity in a cell, the method comprising:
(a) providing a cell having ALT activity and comprising (i) a first DNA tag sequence comprising a first marker, the first DNA tag sequence being integrated into a telomere of a first chromosome of said cell; and (ii) a second DNA tag sequence comprising a second marker, the second DNA tag sequence being integrated into a second chromosome of said cell at a subtelomeric position;
(b) contacting the cell with a test compound;
(c) allowing the cell to undergo division; and
(d) determining in the progeny of the cell whether there is any change in the rate or incidence with which the first DNA tag sequence is copied into a telomere that contains the second DNA tag sequence as compared with an untreated cell.

Preferably, the cell having ALT activity comprises a chromosome that comprises a third DNA tag sequence integrated at an interstitial site, the third DNA tag sequence comprising a third marker.

In one embodiment step (d) comprises determining by PCR amplification the presence of chromosomes which comprise both a first DNA sequence tag and a second DNA sequence tag.

In a preferred embodiment, the first DNA tag sequence comprises a first prokaryotic selectable marker and a first endonuclease recognition site positioned 3' to the first prokaryotic selectable marker; the second DNA tag sequence comprises a second prokaryotic selectable marker and a second endonuclease recognition site positioned 5' to the second prokaryotic selectable marker; and step (d) comprises
(i) recovering nucleic acids from the cell;
(ii) contacting the recovered nucleic acids with one or more endonucleases which cleave the first and second endonuclease recognition sites in both the first and second DNA tag sequences;
(iii) contacting the nucleic acids from step (ii) with an enzyme that catalyses intramolecular ligation of the nucleic acids;
(iv) introducing the nucleic acids from step (iii) into one or more bacterial cells; and
(v) selecting bacterial cells that comprise the first prokaryotic selectable marker and the second prokaryotic selectable marker.

More preferably, the cell having ALT activity comprises a chromosome having a third DNA tag sequence integrated at an interstitial site, the third DNA tag sequence comprising a third prokaryotic selectable marker, the third DNA tag sequence is flanked by endonuclease recognition sites, step (ii) further comprises contacting the recovered nucleic acids with one or more endonucleases that cleave the endonuclease recognition sites flanking the third DNA tag sequence; and step (v) further comprises selecting bacterial cells that comprise the third prokaryotic selectable marker.

Preferably the telomeric sequences positioned 3' to the second DNA sequence tag and integrated at a subtelomeric position comprise a third unique endonuclease recognition site and the method further comprises, prior to step (b), a step of introducing into the cell a third endonuclease which cleaves the third unique endonuclease recognition site in said telomeric sequences.

The sixth aspect of the present invention also provides an assay for screening a compound for an effect on ALT activity in a cell, the assay comprising:
(a) providing a cell having two chromosomes tagged with a detectable marker, the first chromosome having a tag within its telomere and the second chromosome having a tag in a sub-telomeric location;
(b) treating the cell with a test compound;
(c) allowing the cell to undergo division; and
(d) assaying the frequency with which the telomeric tag gets copied into the telomere that contains a sub-telomeric tag.

Preferably, the cell comprises a chromosome that is tagged at an interstitial site to act as an internal assay control.

The assays according to the fifth and sixth aspects of the present invention are particularly suitable for screening for new anti-cancer compounds.

In a seventh aspect, the present invention provides an assay method for determining whether a gene product affects ALT activity in a eukaryotic cell, the method comprising:
(a) providing a cell having ALT activity and comprising a first DNA tag sequence comprising a marker, the DNA tag sequence being integrated into one or more telomeres of said cell;
(b) altering the levels of the gene product in said cell;
(c) allowing the cell to undergo division; and
(d) determining in the progeny of the cell whether there is any change in the rate or incidence with which the first DNA tag sequence is copied into a telomere that contains the second DNA tag sequence as compared with control cells.

In another embodiment, the seventh aspect of the invention provides an assay method for determining whether a gene product affects ALT activity in a eukaryotic cell, the method comprising:
(a) providing a cell having ALT activity and comprising (i) a first DNA tag sequence comprising a first marker, the first DNA tag sequence being integrated into a telomere of a first chromosome of said cell; and (ii) a second DNA tag sequence comprising a second marker, the second DNA tag sequence being integrated into a second chromosome of said cell at a subtelomeric position;
(b) altering the levels of the gene product in said cell;
(c) allowing the cell to undergo division; and
(d) determining whether there is any change in the rate or incidence of telomeric incorporation of the first DNA tag sequence in additional telomeres in the progeny of the cell as compared with control cells.

In one embodiment, the levels of the gene product are altered by introducing into the cell a nucleic acid which is capable of directing expression of the gene product in said cell and incubating the cell under conditions that cause expression of the gene product

Preferably the cell having ALT activity comprises a chromosome that comprises a third DNA tag sequence integrated at an interstitial site, the third DNA tag sequence comprising a third marker.

In one embodiment step (d) comprises determining by PCR amplification the presence of chromosomes which comprise both a first DNA sequence tag and a second DNA sequence tag.

In a preferred embodiment, the first DNA tag sequence comprises a first prokaryotic selectable marker positioned 5' to the first marker and a first endonuclease recognition site positioned 3' to the first prokaryotic selectable marker; the second DNA tag sequence comprises a second prokaryotic selectable marker positioned 3' to the second marker and a second endonuclease recognition site positioned 5' to the second prokaryotic selectable marker; and step (d) comprises
(i) recovering nucleic acids from the cells;
(ii) contacting the recovered nucleic acids with one or more endonucleases which cleave the first and second endonuclease recognition sites in both the first and second DNA tag sequences;
(iii) contacting the nucleic acids from step (ii) with an enzyme that catalyses intramolecular ligation of the nucleic acids;
(iv) introducing the nucleic acids from step (iii) into one or more bacterial cells; and
(v) selecting bacterial cells that comprise the first prokaryotic selectable marker and the second prokaryotic selectable marker.

Preferably the cell having ALT activity comprises a chromosome having a third DNA tag sequence integrated at an interstitial site, the third DNA tag sequence comprising a third prokaryotic selectable marker, the third DNA tag sequence is flanked by endonuclease recognition sites, step (ii) further comprises contacting the recovered nucleic acids with one or more endonucleases that cleave the endonuclease recognition sites flanking the third DNA tag sequence; and step (v) further comprises selecting bacterial cells that comprise the third prokaryotic selectable marker.

In a further preferred embodiment, the telomeric sequences positioned 3' to the second DNA sequence tag integrated at a subtelomeric position comprise a third unique endonuclease recognition site and the method further comprises, prior to step (b), a step of introducing into the cell a third endonuclease which cleaves the third unique endonuclease recognition site in said telomeric sequences.

In the seventh aspect of the invention the nucleic acid which encodes the gene product may be heterologous to the cell.

The seventh aspect of the present invention also consists in an assay for screening whether a gene and/or its expression product affects ALT activity in a eukaryotic cell, the assay comprising:
(a) providing a cell capable of expressing the gene of interest, the cell further having ALT activity and having a plasmid according to the first aspect of the present invention integrated into one or more telomeres;
(b) causing the cell to express the gene;
(c) allowing the cell to undergo division; and
(d) detecting for any change in the rate or incidence of incorporation of the tag from the plasmid in additional telomeres in the cell progeny as compared with the cell not expressing the nucleic acid molecule encoding the gene.

The cell may naturally contain or express the gene of interest or may be altered or engineered by known techniques to contain or express the gene.

A decrease in the rate or incidence of incorporation of the tag is indicative of inhibition of ALT activity by the gene or gene product. An increase in the rate or incidence of incorporation of the tag is indicative of stimulation of ALT activity by the gene or gene product. No change in the rate or incidence of incorporation of the tag is indicative of no effect on ALT activity by the gene or gene product

In an eighth aspect, the present invention relates to a method of introducing a tagged telomere into a cell, the method comprising:
(a) providing a host cell according to the third aspect of the invention which comprises a chromosome having integrated into its telomere a tagged DNA sequence; and
(b) introducing the chromosome into a recipient cell to form a recipient cell comprising a chromosome having integrated into its telomere a tagged DNA sequence.

In another embodiment, the eighth aspect relates to a method of introducing a tagged telomere into a cell, the method comprising:
(a) providing a host cell according to the third aspect of the invention which comprises a chromosome having a tagged DNA sequence integrated at a subtelomeric position; and
(b) introducing the chromosome into a recipient cell to form a recipient cell which comprises a chromosome having a tagged DNA sequence integrated at a subtelomeric position.

The chromosome may for example be introduced by microcell mediated chromosome transfer.

The eighth aspect of the invention also relates to a method of introducing a tagged telomere into a cell, the method comprising:
(a) obtaining a human chromosome comprising a plasmid DNA tag from a cell according to the third aspect of the present invention; and
(b) introducing the chromosome into a cell to form a cell having a tagged chromosome.

The chromosome is preferably introduced by microcell mediated chromosome transfer. It will be appreciated, however, that any means suitable for chromosome transfer between cells would be suitable.

The chromosome can be telomerically or sub-telomerically tagged. A cell produced having a sub-telomeric tagged chromosome is useful as a control as the plasmid does not move to other telomeres by telomere-telomere recombination, regardless of whether the cell has ALT or not.

In a ninth aspect, the present invention relates to a method of removing a distal part of a telomere in a cell, the method comprising:
(a) providing a nucleic acid construct according to the second aspect of the invention, which construct comprises within the telomeric multiple repeats a third unique endonuclease recognition site;
(b) transfecting the cell with the nucleic acid construct;
(c) incubating the cell to allow the nucleic acid construct to integrate into a chromosome of the cell at a subtelomeric position; and
(d) introducing into the cell an endonuclease which cleaves the third endonuclease recognition site.

Another embodiment of the ninth aspect of the invention provides a method of removing a distal part of a telomere in a cell, the method comprising:
(a) modifying a plasmid according to the first aspect of the present invention by inserting a recognition site for an endonuclease adjacent to the tag;
(b) transfecting a cell with a linearised modified plasmid to form a modified cell having a tag which is immediately sub-telomeric in a chromosome; and
(c) treating the cell with an endonuclease to cleave the telomere from any chromosome containing the endonuclease cleavage site to form a cell having a distal part of a telomere removed.

Preferably the endonuclease is HO endonuclease.

In a tenth aspect, the present invention relates to use of the plasmids/nucleic acid constructs according to the first or second aspects of the present invention in assays for ALT activity in eukaryotic cells.

In an eleventh aspect, the present invention provides a nucleic acid vector which comprises, and/or when linearised comprises, a nucleic acid construct according to the first or second aspects of the invention. Preferably pSXneo-1.6T₂AG₃ described in Hanish et al., 1994 is specifically excluded.

In a twelfth aspect, the present invention provides a method of producing a nucleic acid construct of the first or second aspect of the invention which method comprises linearising a nucleic acid vector of the eleventh aspect of the invention. Preferably pSXneo-1.6T₂AG₃ described in Hanish et al., 1994 is specifically excluded.

In a thirteenth aspect, the present invention provides a kit comprising nucleic acid vectors which comprise, and/or when linearised, comprise nucleic acid constructs of the first and second aspects of the invention. The kit may be used in a method of assaying for ALT activity in a eukaryotic cell.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any description of prior art documents herein is not an admission that the documents form part of the common general knowledge of the relevant art in Australia.

### Detailed description of the invention

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook *et al*., Molecular Cloning: A Laboratory Manual, 2^{nd} ed. (1989) Cold Spring Harbor Laboratory Press, Cold Spring Harbor, N.Y. and Ausubel *et al*., Short Protocols in Molecular Biology (1999) 4^{th} Ed, John Wiley & Sons, Inc. - and the full version entitled Current Protocols in Molecular Biology, which are incorporated herein by reference) and chemical methods.

References to 5' and 3' in relation to chromosomes and sequences integrated therein are to be interpreted as follows. The 5' end is the end nearest to the centromere. The 3' end is the end nearest to the telomere end.

### Nucleic acid constructs

Nucleic acid constructs of the invention are typically double-stranded DNA. They are capable of integrating into eukaryotic chromosomes at a telomeric or sub-telomeric position by virtue of comprising multiple repeats of , telomeric sequences such as human telomeric sequences or sequences homologous to human telomeric sequences. In general, the telomere multiple repeats will be selected so as to be compatible with the chromosome into which the nucleic acid constructs are designed to integrate. For example, when the constructs are intended to be integrated into human chromosomes, preferred telomeric repeat sequences are human telomere repeats, namely (TTAGGG)n - specified in the 5' to 3' direction from the centromere toward the telomere end. Other types of telomere repeats have been identified in other eukaryotic cells such as yeast cells.

The number of telomeric multiple repeats should be so as to provide a sufficiently large region homologous to the target chromosome to permit homologous recombination between the target chromosome and the nucleic acid construct to occur. For example, the multiple repeat sequence may comprise at least 500, 800 or 1000 base pairs.

Nucleic acid constructs may be circular DNA molecules (e.g. plasmids) or linear DNA molecules. It is preferred that the nucleic acid constructs are linear prior to integration since linear DNA molecules are more recombinogenic. Circular DNA molecules may, for example, be linearised prior to introduction into eukaryotic cells by cutting with restriction endonucleases as described below.

Nucleic acid constructs of the invention also comprise a DNA tag sequence comprising a marker to enable them to be detected by a variety of means, typically hybridisation based procedures such as in situ-hybridisation, polymerase chain reaction and/or Southern blotting. The marker is typically a selectable marker, such as an antibiotic resistance gene suitable for use in eukaryotic cells. When the cells are being grown for several generations, growth of the cells under selection pressure assists in maintaining cells with tagged telomeres in the cell population.

The marker will comprise the necessary transcriptional/translational control sequences to permit expression of the selectable marker in a eukaryotic cell. Suitable eukaryotic resistance genes include genes encoding neomycin, puromycin or hygromycin resistance.

The nucleic acid constructs of the first aspect of the invention comprise a first DNA tag sequence and are designed to integrate the tag within the telomeres of a eukaryotic chromosome. Accordingly, the first DNA tag sequence is flanked by two arms of telomeric sequence repeats, a first DNA sequence positioned 3' of the tag (which when integrated will be nearest the telomere end of the chromosome) and a second DNA sequence positioned 5' of the tag (which when integrated will be the other side of the tag, towards the centromere of the chromosome).

The nucleic acid constructs of the second aspect of the invention comprise a second DNA tag sequence and are designed to integrate the tag at a sub-telomeric position within a eukaryotic chromosome. This means that when integrated, there should essentially be no telomeric repeat sequences between the second DNA tag sequence and the centromere - the only telomeric repeat sequences should be positioned 3' of the second DNA tag sequence, towards the end of the chromosome. Accordingly, the second DNA tag sequence is linked to a first DNA sequence positioned 3' of the tag which comprises multiple telomere repeats (which when integrated will be nearest the telomere end of the chromosome) and optionally a third DNA sequence positioned 5' of the tag, such that any DNA sequence 5' of the tag sequence does not contain telomeric repeats.

The nucleic acid constructs of the second aspect of the invention typically cause truncation when they integrate into a chromosome. However, the third DNA sequence may be selected to include sequences homologous to sequences present in a sub-telomeric region of a target chromosome. This may reduce the possibility of truncation.

The second DNA tag sequence comprises a second marker. Generally, the second marker will be distinguishable from the first marker so that detection of the markers (and/or selection for the markers as appropriate) can be used to determine whether a chromosome has a tag integrated at a telomeric or sub-telomeric position, or both. For example, the first marker may be a first eukaryotic selectable marker such as a gene encoding neomycin resistance and the second marker may be a second eukaryotic selectable marker such as a gene encoding hygromycin resistance.

In one embodiment of the present invention, the DNA tag sequences further comprise, in addition to a eukaryotic selectable marker, a prokaryotic selectable marker, and optionally, a bacterial origin of replication. These allow the nucleic acid constructs to be propagated in prokaryotic cells.

In a particular embodiment of the present invention, the first and second DNA sequence tags also comprise endonuclease recognition sites such that when a eukaryotic chromosome comprises both a first tag and a second tag, the DNA sequence tags can be recovered and separated from the chromosomal DNA and propagated in prokaryotic cells, as described below. Specifically, the first DNA sequence tag is configured such that a first endonuclease recognition site is positioned 3' of the first prokaryotic selectable marker (which integrates within the telomere) and the second DNA sequence tag is configured such that a second endonuclease recognition site is positioned 5' of the second prokaryotic selectable marker (which integrates at a subtelomeric position).

The endonuclease recognition sites should be chosen so that there are no other equivalent sites present between the telomere end and the 5' end of the second prokaryotic selectable marker. This is to prevent cleavage within or between the prokaryotic selectable markers when the appropriate endonucleases are used to excise the region containing both prokaryotic selectable markers from the eukaryotic chromosomal DNA. It is less important whether the endonucleases cleave other parts of the chromosome or other chromosomes. However, there may be advantages to using endonuclease recognition sites that are unique in the sense that the only sites present are those in the desired location within the DNA tag sequences.

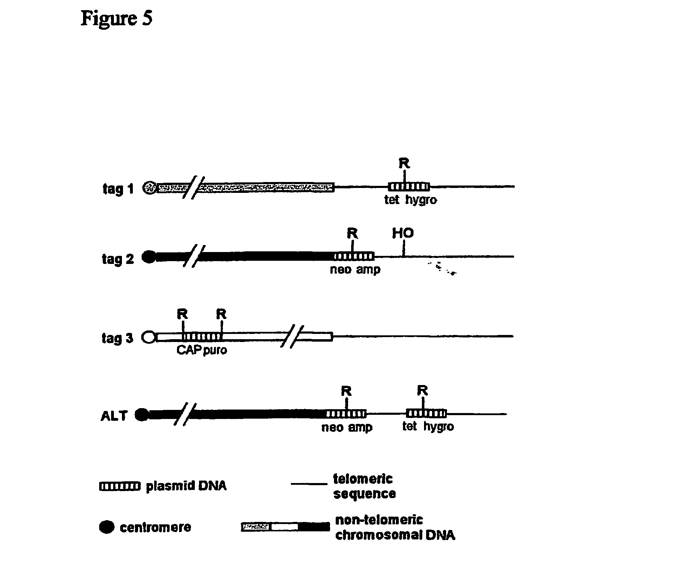

An example of a suitable configuration, when integrated into the chromosomal DNA, is shown in Figure 5, the endonuclease recognition sites being depicted as "R". Cleavage at both R sites in the ALT chromosome will result in a fragment containing both an ampicillin resistance gene and a tetracycline resistance gene, and optionally a bacterial origin of replication.

The two endonuclease recognition sites may be the same or different. Examples of endonuclease recognition sites include a recognition site for a type II restriction endonuclease. Preferably the type II restriction endonuclease is a "rare cutter", for example having a cognate recognition site comprising at least 8 defined nucleotides.

In certain embodiments, it may be desirable to include in the multiple sequence repeats of the nucleic acid constructs of the second aspect of the invention, a third endonuclease recognition site. Generally, the third endonuclease recognition site is not cleaved by endonucleases that recognise the first and second endonuclease recognition sites. Preferably the endonuclease recognition site sequence is chosen so as to be unique to the genome of the cell, i.e. is not present elsewhere in the genome of the host cell. A preferred example of a nuclease which cleaves at the third endonuclease recognition site is HO endonuclease.

### Nucleic acid vectors

Nucleic acid constructs of the present invention can be incorporated into recombinant nucleic acid vectors. The vector may be used to replicate the nucleic acid in a compatible host cell. Suitable host cells include bacteria such as *E*. *coli,* yeast, mammalian cell lines and other eukaryotic cell lines. Typically, the vector is a plasmid vector that can be replicated in a prokaryotic host.

Where nucleic acid constructs of the invention are used in linear form but are present in a circular plasmid, it is generally desirable to linearise the plasmid DNA with endonucleases prior to introduction into a target eukaryotic cell. In some circumstances, the final configuration required is only achieved after the plasmid is linearised. For example, a nucleic acid construct according to a first aspect of the invention comprises telomeric repeats flanking the first DNA tag sequence. However, such a construct may be obtained from a plasmid construct wherein both sets of repeats are present on the same end of the tag sequence by cleaving the plasmid within the multiple repeats. The resulting linear nucleic acid construct is then in the correct configuration.

Accordingly, nucleic acid vectors of the invention may either comprise a nucleic acid construct of the invention as defined above or may be capable of producing a nucleic acid construct of the invention when treated appropriately, e.g. when linearised by complete digestion with a restriction enzyme.

### Host cells

Suitable host cells may be either eukaryotic cells, such as yeast or mammalian cells, or prokaryotic cells, such as bacterial cells. Eukaryotic cells comprising nucleic acid constructs of the invention integrated into one or more chromosomes are typically used to assay for ALT activity whereas prokaryotic cells are used to propagate constructs for subsequent use, or in one aspect to quantify the results of ALT activity determinations in eukaryotic cells.

Preferably eukaryotic host cells have ALT activity. Human cells are also preferred.

Host cells include mammalian cell lines and cells obtained from a human or animal, such as suspected cancer cells.

Nucleic acid constructs of the invention may be introduced into host cells using suitable means known in the art. In the case of eukaryotic cells, suitable techniques include transfection using transfection agents including cationic agents (for example calcium phosphate and DEAE-dextran) and lipofectants (for example lipofectam™ and transfectam™). Typically, nucleic acid constructs are mixed with the transfection agent to produce a composition.

Following transfection, eukaryotic cells are typically grown in the presence of an agent that selectively inhibits growth of cells that do not comprise a nucleic acid construct of the invention e.g. an antibiotic. This allows selection of successfully transfected cells since only cells comprising a nucleic acid construct of the invention can grow in the presence of the antibiotic. When cells are transfected with a nucleic acid construct according to the second aspect of the invention it may be desirable to check the location of the second DNA tag sequence by techniques such as FISH or Southern blotting to ensure that the second DNA tag sequence has integrated at a sub-telomeric location.

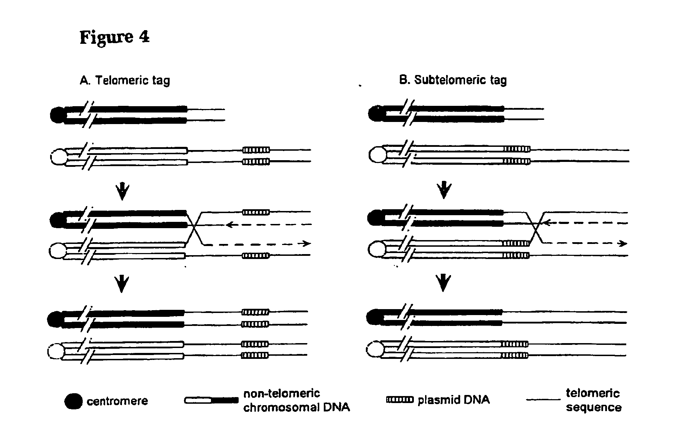

As a result of transfection and selection, eukaryotic host cells may be obtained which comprise integrated into one or more chromosomes a first DNA tag sequence and/or a second DNA tag sequence. The first tag is integrated at a telomeric position and the second tag is integrated at a subtelomeric position (see Figures 4, 5 and 6, for example). In some embodiments, it may be desirable to produce host cells that comprise a chromosome having two first DNA tag sequences integrated telomerically. In these cases, the two first DNA tag sequence should have different marker sequences.

In one embodiment, host cells of the invention comprise a chromosome comprising a first tag, a chromosome comprising a second tag and a chromosome comprising a third tag, where the third tag is integrated at an interstitial position in the chromosome. The chromosome comprising the third tag may be on the same or a different chromosome to the first or second tags. Preferably the third tag comprises a third selectable eukaryotic marker, a third prokaryotic selectable marker, both different from the first and second eukaryotic/prokaryotic markers, the third prokaryotic selectable marker being flanked by unique endonuclease recognition sites which allow the third prokarytic selectable marker to be recovered from the chromosomal DNA and cloned into a prokaryotic host cell (see Figure 5). The third tag sequence may be used as a control in certain configurations of ALT activity assays described below.

The presence of tag sequences may be confirmed by detecting the presence of the markers in the tag sequences, using for example in-situ hybridisation or PCR/Southern blotting of genomic DNA.

Eukaryotic host cells of the present invention may be used in assays for ALT activity, for example as described below.

They may also be used to introduce tagged telomeres into other host cells. Typically, the process involves transferring a chromosome comprising a tagged telomere from a donor host cell of the invention to a recipient cell using standard techniques. Preferably the recipient cell has telomerase activity.

An example of a suitable technique is microcell-mediated chromosome transfer in which the donor cell is fragmented in such a way that individual chromosomes or small groups of chromosomes become surrounded by membrane which is then fused to the "recipient" cell line. In the case of human donor host cells and mouse recipient cells, the resulting hybrid nucleus containing mouse chromosomes plus one, or a few, human chromosomes, progressively and randomly lose human chromosomes. Cells that retain a chromosome comprising a DNA tag sequence can be selected on the basis of selectable marker sequences, such as eukaryotic antibiotic resistance genes. These recipient cells may be used as such or they may be used as a source of tagged chromosomes for transfer into further recipient cells. For example the human chromosome present in the mouse/human hybrid may be transferred into a human cell, using techniques such as microcell-mediated chromosome transfer. Selection for the marker present in the tagged human chromosome may be used to exclude recipient cells that contain only mouse chromosomes.

Host cells of the invention can, for example be used to test candidate ALT repressor genes, to test candidate genes for activation of ALT, and to screen compounds for their ability to act as ALT inhibitors. Suitable assays are described below.

### ALT assays

The present invention provides assays for alternative lengthening of telomeres (ALT) activity in eukaryotic cells, such as mammalian cells including human cells. The basis for the assay is the introduction of a marker sequence into one or more telomeres of the cell by homologous recombination, typically using a nucleic acid construct of the first aspect of the invention which integrates within the telomere. Cells that have integrated the nucleic acid construct are then allowed to divide. If ALT activity is present in the cell then inter-telomeric templating may occur, resulting in copying of the marker sequence from a tagged chromosome to another chromosome by homologous recombination. This type of event can be measured by detecting the presence and optionally the amount of marker sequence in cells. The marker may be detected by a variety of means, typically hybridisation based procedures such as in situ-hybridisation, polymerase chain reaction and/or Southern blotting. A variety of configurations may be used, a number of which are described in more detail below.

### ALT Assay #1

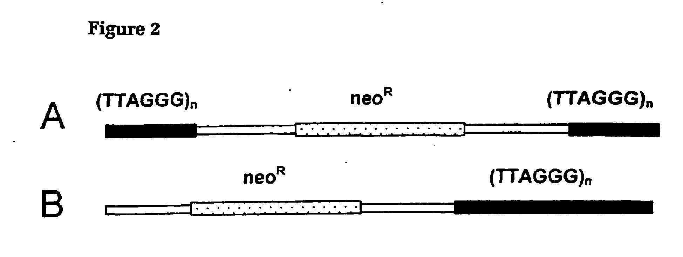

A nucleic acid construct of the first aspect of the invention that contains two arms of telomeric DNA sequence (i.e., DNA that contains multiple telomere repeats such as repeats of the TTAGGG hexanucleotide found in human telomeres) on either side of non-telomeric plasmid DNA sequence (referred to as the "tag") that includes a eukaryotic selectable marker gene (Figure 2A), is introduced into a cell, for example by transfection. This construct is typically produced by linearising a suitable plasmid (construction of an example of such a plasmid is described below in the Examples - Materials and Methods section). The construct is designed to integrate into telomeric DNA via homologous recombination.

Colonies of cells that have integrated the construct are selected using the selectable marker. Colonies are then examined to determine which ones have integrated the construct into one or more telomeres. Methods of examination include fluorescence in situ hybridisation (FISH) using a probe for the DNA tag sequence. Those colonies that contain one or more telomeric integrations are cultured further, and examined again by FISH to determine whether the tag is now present on additional telomeres (Figure 4, panel A). If this has occurred more often than in telomerase-positive cells, then the cell has ALT activity. For example, where the telomerase-positive cells show substantially no tags on additional telomeres then additional tags in test cells will be indicative of ALT activity. However, where there is a low level of additional tags in telomerase-positive cells then there will need to be additional tags above this background level in test cells for them to be considered ALT positive.

As a control, the cells may also be transfected with a second nucleic acid construct according to the second aspect of the invention that is designed to integrate in such a way that it is immediately proximal to a telomere. This construct has telomeric DNA 3' to the non-telomeric DNA tag sequence (Figure 2B). This construct randomly integrates, leading to chromosome breakage, loss of distal chromosome sequence, and then seeding of a new telomere. The final result is that the DNA tag sequence is immediately proximal (centromeric) to the new telomere. In a cell with ALT activity, the tag stays only in its original location and does not become copied into other telomeres, because there is no telomeric repeat DNA available for homologous recombination proximal (centromeric) to the tag (Figure 4, panel B). Typically, the marker within this second construct is a different marker to the one present in the first construct.

### ALT Assay #2

A human chromosome containing a first DNA tag sequence within a telomere, obtained as described for ALT Assay #1, is placed into a telomerase-positive mouse cell line, e.g. A9, by the technique of microcell mediated chromosome transfer. The human chromosome can be distinguished from the mouse chromosomes using probes specific for human or mouse genomic sequences. To assay for ALT activity in a human cell line, the telomerically tagged chromosome is transferred from A9 cells into the human cells, again via microcell mediated chromosome transfer. If the tag can be detected in the telomere of a chromosome other than the one provided by the A9 cell, then that cell line has ALT activity.

As a control, an A9 cell containing a human chromosome with a second DNA tag sequence in a sub-telomeric location is used as the chromosome donor. This second tag does not move to other telomeres by telomere-telomere recombination, regardless of whether the cell line has ALT activity or not.

### ALT Assay #3

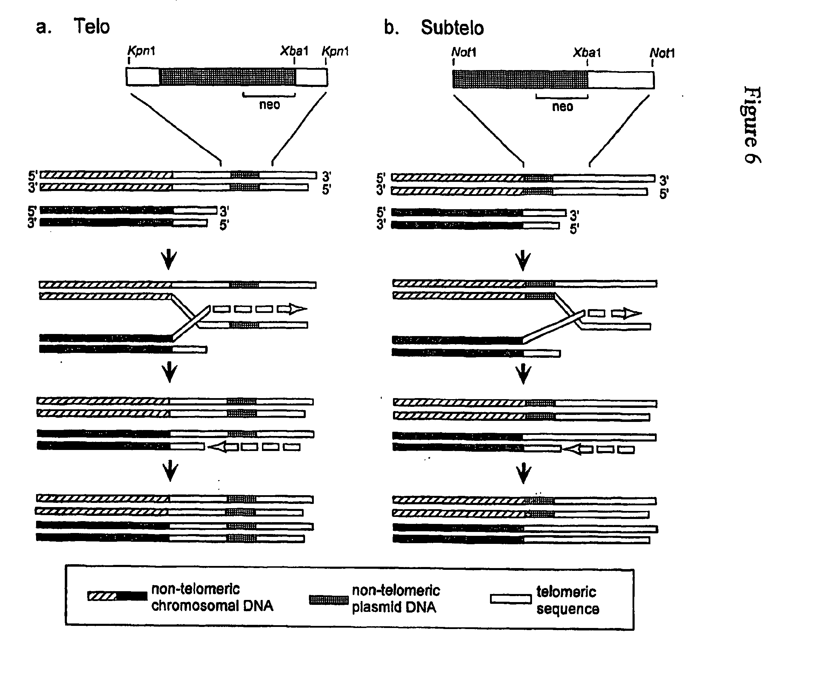

The principle of this assay is that two chromosomes are tagged within a single cell (one within a telomere and another at a sub-telomeric location) and the frequency with which the telomeric tag gets copied into the telomere that contains a sub-telomeric tag acts as an indicator of ALT activity (Figures 4 and 6). Optionally, a chromosome may be tagged at an interstitial site to act as an internal assay control.

The two tags may be designed such that only when they are both present in the same chromosome, PCR of the genomic DNA will amplify a fragment i.e. the presence of the first DNA sequence tag and the second DNA sequence tag in the same chromosome will result in the ability to detect a PCR product. Consequently, ALT activity may be detected by PCR of genomic DNA from progeny cells using suitable primers.

In a particular embodiment of this assay, the measurement of whether the first tag and second tag are present on the same chromosome is carried out by extracting genomic DNA from the eukaryotic cells, digesting the genomic DNA with specific endonucleases and cloning the digested DNA into a prokaryotic host where selection for specific markers is performed. In this embodiment, the first and second DNA tag sequences also comprise different selectable prokaryotic markers. Furthermore, restriction endonuclease recognition sites are present within the first and second DNA tag sequences such that when a eukaryotic chromosome which contains both tags is digested with one or more appropriate endonucleases, a fragment is produced which contains both prokaryotic markers. The configuration required for the nucleic acid constructs and tagged chromosomes is described in detail above in the section relating to nucleic acid constructs.

When the fragment is subcloned into a prokaryotic host cell, the host cell will carry both selectable markers and can be selected accordingly. If the fragment also comprises a bacterial origin of replication, it may simply be ligated (if the ends of the fragment are compatible) and introduced into prokaryotic cells using standard transformation procedures, and grown on selective media. Otherwise, the fragment may be cloned into a prokaryotic vector which is then introduced into prokaryotic host cells. The number of colonies growing on the selective medium gives an indication of the extent to which the eukaryotic cells contained doubly tagged chromosomes.

As a control, a third tag is introduced into the eukaryotic cells which comprises a third prokaryotic selectable marker and is flanked by endonuclease recognition sites which allow the marker to be recovered from the chromosomal DNA and subcloned into a prokaryotic host in a similar manner.

A specific example of this assay is as follows:
(i) The sub-telomeric tag (tag 2) contains a neoR gene, and a beta-lactamase ampicillin resistance gene; when linearised with a restriction enzyme, it has (TTAGGG)n sequence distal to the remainder of the plasmid DNA. This construct is optionally modified by the insertion of a recognition site for HO endonuclease (which does not normally cut at any location within the human genome) within the (TTAGGG)n sequence. ALT cells are transfected with the linearised plasmid, and G418-resistant colonies are then screened by FISH using a neoR probe. Those colonies that appear to contain a single sub-telomeric tag are analysed further by Southern blotting and DNA sequencing to confirm that the tag is indeed immediately sub-telomeric. One such clone is further modified as follows.
(ii) The telomeric tag (tag 1) contains hygromycin resistance (eukaryotic selectable marker) and tetracycline resistance (prokaryotic marker); when linearised there is (TTAGGG)n sequence flanking the remainder of the tag sequence (i.e., the non-TTAGGG sequence). This plasmid is transfected into the cells produced as described in (i). Hygromycin-resistant colonies are screened by FISH to identify those that appear to contain only telomeric tags (i.e. tag 1 in a telomeric location), and these are subjected to further molecular analyses for confirmation. One clone is then modified as follows.
(iii) An interstitial tag (tag 3) is inserted by transfecting the cells with a construct containing a puromycin resistance gene (eukaryotic selectable marker) and chloramphenicol resistance (prokaryotic marker), but contains no telomeric sequence, and then selecting for puromycin resistance. Although the HO endonuclease site has been specified, other analogous sites such as that for SceI could be substituted. Also, the three tags can be introduced into the cell in other orders.

Additional features of the plasmids are as follows. Firstly, the plasmids contain one copy (tags 1 and 2) or two copies (tag 3) of a restriction site R (Figure 5), such that apposition of tag 2 to tag 1 within the cell by ALT activity will result in the ability to recover a plasmid with dual prokaryotic resistance markers. The same procedure will recover tag 3 as an internal assay control.

Secondly, they may contain sequences suitable for PCR amplification such that apposition of tag 2 to tag 1 within the cell by ALT activity will result in the ability to detect a PCR product The PCR reaction may also detect a product from the integrated tag 3 as an internal assay control. As mentioned above, this system may also be used without the need for prokaryotic markers.

From the time the telomeric tag is inserted into the cells as described in (ii), it will be copied on to other telomeres which may include the one that contains the sub-telomeric tag. Where sub-telomerically tagged chromosomes also comprise within the telomeric sequences a third unique endonuclease recognition site, then it is preferred immediately before carrying out an assay, for telomeric tags to be excised from the chromosome with the sub-telomeric tag by treating the cells with recombinant endonuclease, such as HO endonuclease. The recombinant endonuclease may be modified to contain the protein transduction domain from the human immunodeficiency virus TAT protein that will enable it to penetrate the nucleus with high efficiency. (Other nucleases may be used; other protein transduction domains may be used; or the protein transduction technique may be replaced by expressing the endonuclease under the control of an inducible promoter).

At later time points, copying of the telomeric tag to this chromosome will be measured by extracting genomic DNA, cutting with restriction enzymes as indicated (Figure 5), religating with T4 DNA ligase, transforming competent *E*. *coli,* and plating out on agar containing either ampicillin plus tetracycline, or chloramphenicol alone. The number of colonies on the amp/tet plates will be proportional to the number of recombination events that bring the telomeric tag sufficiently close to the sub-telomeric tag to permit recovery of a plasmid with both prokaryotic markers. Chloramphenicol-resistant colonies are due to recovery of the interstitial tag, and the number of these colonies will be the internal control of assay efficiency in all assays performed. Alternatively, PCR may be used to detect the apposition of tags 1 and 2.

In a modification of ALT assay #3, two telomeric tags (i.e. first DNA tag sequences) which have a different marker sequence are introduced into the same chromosome. Copying of both tags to another chromosome can be detected using the same techniques as described above in ALT assay #3, i.e. PCR using primers that only produce a PCR fragment when the two different first tags are in the same chromosome, or by recovering genomic DNA and subcloning into a prokaryotic host.

### Uses of the ALT assay

An assay of ALT activity according to the present invention can, for example, be used for the following purposes:
a) determining whether immortalised cells utilise the ALT telomere maintenance mechanism;
b) determining whether a gene and/or its expression product(s) increase or decrease ALT activity;
c) determining whether a compound is able to inhibit ALT activity (i.e., to screen for ALT inhibitor drugs, such as anti-cancer drugs).

### Assays for compounds/gene products which affect ALT activity

In addition to measuring ALT activity in cells, such as tumour cells, the nucleic acid constructs and host cells of the invention may be used to screen compounds and gene products for an effect on ALT activity.

Essentially, these assays are performed in a similar manner to those described above, except that measurements of ALT activity are performed in the presence and absence of a test compound or gene product

Host cells are provided which contain at least a first DNA tag sequence integrated within one or more telomeres. One sample of cells (the control) is then cultured, and the rate and/or extent of copying of the first DNA tag to other chromosomes measured as described above. Another sample of cells is also cultured but in the presence of a candidate compound. Again the rate and/or extent of copying of the first DNA tag to other chromosomes is measured. The results in the presence of the compound are then compared with the control. If an increase is obtained then the compound is considered to stimulate ALT activity, if a decrease is obtained then the compound is considered to be an inhibitor of ALT activity.

In a preferred embodiment, the host cells also comprise at least a second DNA tag sequence integrated at a sub-telomeric position. Typically, ALT activity is measured by determining whether a first DNA tag is copied onto a chromosome comprising a second DNA tag. This may be achieved using the techniques described in ALT assay #3, i.e. by PCR for juxtaposed tags or by extracting genomic DNA, digesting and subcloning into a prokaryotic vector as described above.

Candidate compounds include topoisomerase inhibitors or compounds such as modified oligonucleotides that reduce the expression of genes involved in ALT, and may also include antibody products (for example, monoclonal and polyclonal antibodies, single chain antibodies, chimeric antibodies and CDR-grafted antibodies). Furthermore, combinatorial libraries, peptide and peptide mimetics, defined chemical entities, oligonucleotides, and natural product libraries may be screened for activity as modulators of ALT activity. The candidate substances may, for example, be used in an initial screen in batches of, for example 10 substances per reaction, and the substances of those batches which show inhibition tested individually.

Candidate compounds are typically added to a final concentration of from 1 to 1000 nmol/ml, more preferably from 1 to 100 nmol/ml. In the case of antibodies, the final concentration used is typically from 100 to 500 µg/ml, more preferably from 200 to 300 µg/ml.

The above screening methods may also be used to screen gene products for an effect on ALT activity. The levels of a gene product in a host cell are altered (by which is typically meant the levels of functional gene product rather than just the actual number of molecules of gene product present in the cell) and the effect on ALT activity determined. For example, the levels of the gene product may be altered by introducing into the cell a nucleic acid which is capable of directing expression of the gene product in the cell and incubating the cell under conditions that cause expression of the gene product. The nucleic acid sequence (which may be DNA or RNA) may be introduced into the host cells using standard techniques such as transfection or direct injection. The nucleic acid sequence may be present in both the control cells and the test cells, or only the test cells. Where the nucleic acid sequence is present in the control cells, the coding sequence may be linked to an inducible promoter so that expression of the gene product can be induced in the test cells.

The nucleic acid sequences may be heterologous to the cell. The term "heterologous to the cell" means that the sequences are not part of the normal cell genome.

However, the levels of gene product may be altered using other techniques. For example, a gene product (such as a protein) may be injected directly into the cell. Alternatively, compounds/nucleic acids may be introduced into the cell which affect the levels of expression of an endogenous gene product (for example an antisense construct, an interfering RNA, a dominant negative construct, a ribozyme or an antibody).

Nucleic acid sequences of interest may, for example, be specific known sequences, or unknown sequences obtained from a library of sequences. Thus, for example, the assays of the invention may be used to test a known gene product for an effect on ALT activity or to screen a library of sequences for gene products that affect ALT activity.

### Use of ALT inhibitors

ALT inhibitors, such as those identified by the assay methods of the invention (in combination with telomerase inhibitors) may potentially be used for the short term and/or long-term treatment of any cancer that is not cured by surgery, or where there is a risk of relapse after surgery (more than 50% of all cancers may fall into these categories). It is also possible that combination telomere maintenance inhibitors may be used for prevention of cancer in high-risk individuals.

Inhibition of telomere maintenance in immortalised cells results in telomere shortening followed by cessation of growth. Because of the number of population doublings this takes (the number being proportional to the initial telomere length, which varies among cancers), telomere maintenance inhibitors are unlikely to be used for tumour debulking (i.e., initial treatment) and are more likely to be used to prevent regrowth of tumours from the small number of cells remaining after other forms of treatment (i.e., adjuvant therapy).

Normal cells do not appear to be dependent on telomere length maintenance mechanisms. It thus seems likely that inhibitors of telomere maintenance will have minimal toxicity for normal cells. They may therefore be suitable for long-term treatment designed to prevent recurrence of cancer or even, in some cases, for cancer prevention in individuals with a high risk of cancer.

In order that the present invention may be more clearly understood, the present invention will now be described with reference to the following examples and drawings, which are illustrative only and non-limiting.

### Detailed Description of Drawings

Figure 1. A model for inter-chromosomal recombination-mediated lengthening of telomeres.
   (a) For various reasons, including when it becomes critically short, a telomere may be interpreted by the cell as a double strand break (DSB). (b) The DSB repair enzymes then mediate invasion by a single-stranded 3' end of the short telomere between the strands of a longer telomere. This step may be dependent on RAD52. DNA polymerase may then extend the short strand, using the long strand as the template. (c) The crossed-over strands may then be subject to cleavage by a nuclease (→) followed by ligation, resulting in recombinant DNA molecules (d). Alternatively, the structure shown in (b) may be resolved by unwinding of the newly formed helix and rewinding (c), resulting in non-recombinant molecules (d). In either case, the staggered annealing of repeats in the short and long telomeres results in net telomere elongation. From (Reddel et al, 1997).
Figure 2. Plasmids used for an ALT assay #1 according to the present invention.
   (A) Plasmid designed to integrate into a telomere by homologous recombination. When linearised, the plasmid has two arms of (TTAGGG)n DNA (black bars) flanking non-TTAGGG plasmid "tag" DNA (white bar) that includes a bacterial origin of replication, a bacterial selectable marker, and a selectable marker designed for expression in eukaryotic cells (stippled bar; the neomycin resistance gene is illustrated, but any suitable eukaryotic marker could be used).
   (B) Plasmid designed to integrate in a sub-telomeric position. When linearised, this plasmid has (TTAGGG)n sequence (black bar) 3' to the non-TTAGGG "tag" DNA (white bar).
Figure 3. Map of Tel plasmid. The Kpn1 restriction site of pSXneo-1.6-T₂AG₃ was destroyed at site 2 enabling the plasmid to be linearised using Kpn1 (at site 1) and used as a targeting vector.
Figure 4. ALT assay #1 according to the present invention. ALT involves telomere lengthening via homologous recombination and copying of one telomere by another. If a cell has ALT activity, then a DNA sequence tag placed within a telomere (panel A) will be copied from one telomere to another, but a tag placed immediately proximal to the telomere, i.e. subtelomerically (panel B), will not be copied.
Figure 5. ALT assay #3 according to the present invention. In this assay system, two or three chromosomes are tagged with plasmid DNA: **tag 1** is within one or more telomeres, **tag 2** is immediately sub-telomeric, and **tag 3** is located interstitially (within any chromosome) (tag 3 could be on the same chromosome as tags 1 or 2). Each tag has a different prokaryotic marker - ampicillin (amp), tetracycline (tet) or chloramphenicol (CAP) resistance - and a different eukaryotic marker - neomycin (neo), hygromycin (hygro) or puromycin (puro) resistance. **ALT** activity may result in tags 1 and 2 being located at the same telomere, in which case cutting genomic DNA at the indicated restriction site **(R),** following by transformation of bacteria with the religated and circularised DNA will result in bacteria that are doubly amp and tet resistant, and others (internal control) that are CAP resistant. Background will be reduced by cutting the telomere containing tag 1 with **HO** endonuclease immediately before commencement of the assay.
Figure 6. Strategy for detecting inter-telomeric copying of DNA. α. Inter-telomeric recombination proximal to an integrated telomere targeting plasmid, Tel, will result in copying of the plasmid DNA "tag" sequences to another telomere. ***b***. If Subtel plasmid DNA is located immediately proximal to telomeric DNA, inter-telomeric recombination can only occur distal to the plasmid and the tag will not be copied onto the other chromosome.

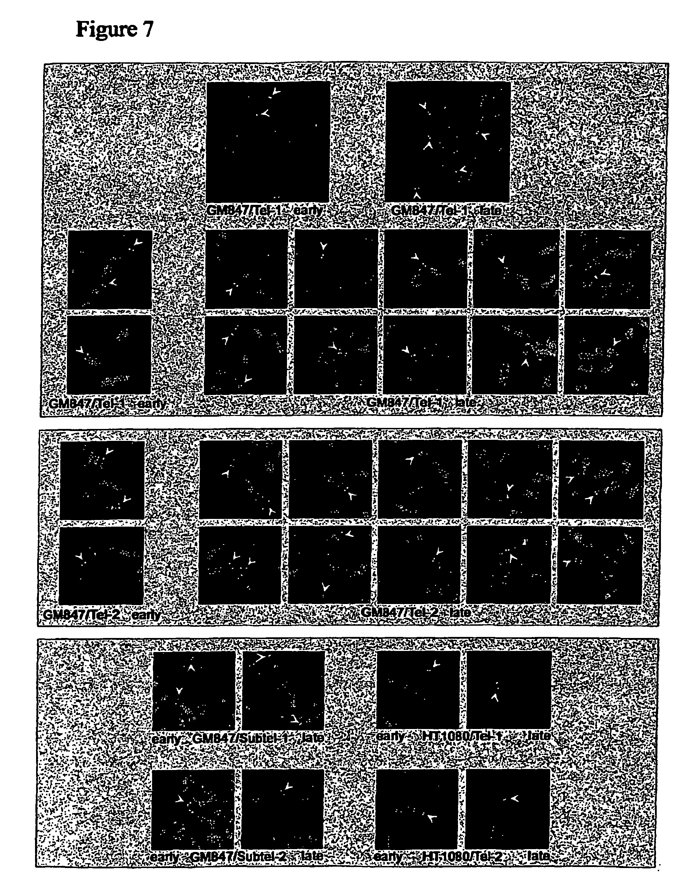

Figure 7. Detection of plasmid tag DNA at the telomere by FISH. Upper and middle panels: full or partial metaphase spreads of GM847/Tel-1 (upper) and GM847/Tel-2 (middle), which are clones of the ALT cell line, GM847, transfected with the telomere targeting plasmid, Tel. Both clones are shown at PD23 (early) and PD63 (late). Lower panel: partial metaphase spreads of GM847/Subtel-1 at PD24 (early) and PD64 (late); GM847/Subtel-2 at PD24 (early) and PD64 (late); HT1080/Tel-1 at PD23 (early) and PD63 (late); HT1080/Tel-2 at PD23 (early) and PD63 (late). The tagged telomeres are described in Table 1.

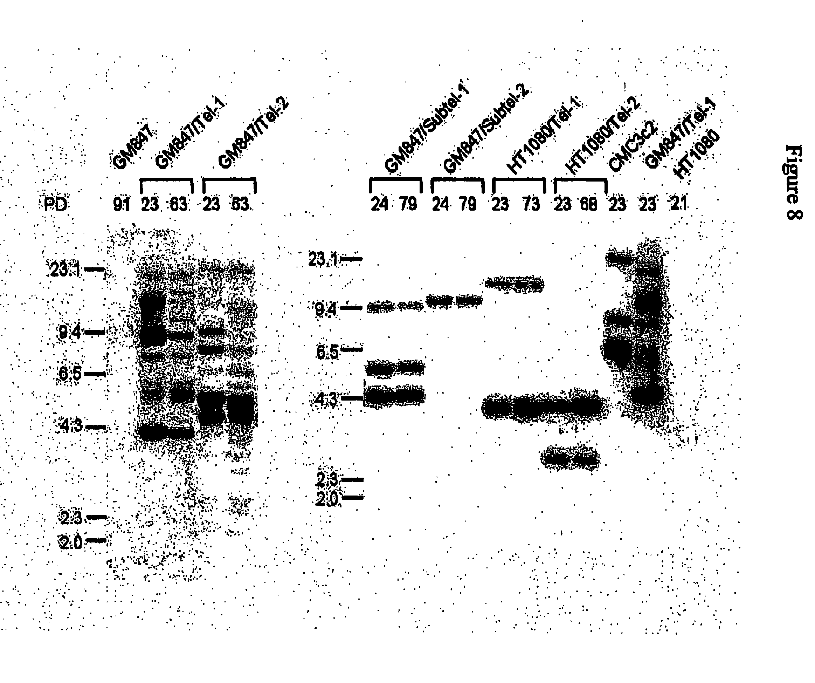

Figure 8. Southern analysis of GM847 and HT1080 lines. Genomic DNA was isolated from the cells at the indicated population doubling (PD), digested with *Xba*I, electrophoresed in 0.8% agarose, Southern blotted and hybridised to radiolabeled α-32P-pSXNeo probe which detects the core plasmid sequences of Tel and Subtel. CMC3c2 is a mouse A9 cell line containing a single tagged human chromosome microcelled from GM847/Tel-1. Size of *Hin*dIII-digested lambda DNA markers is shown in kb

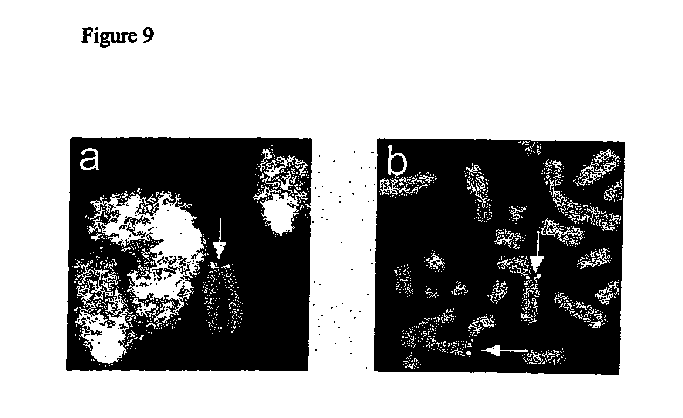

Figure 9. A. A partial metaphase from the CMC3c2 microcell hybrid donor, which contains a telomere tagged human chromosome (arrow) from GM847/Tel-1. The mouse chromosomes have been hybridised with biotinylated mouse Cot-1 DNA (FTTC). B. A partial metaphase from GM847 cells containing the tagged donor chromosome (vertical arrow) transferred from CMC3c2, and a chromosome that has a newly acquired telomere tag (horizontal arrow).

### EXAMPLES

### MATERIALS AND METHODS

### Construction of Tel Plasmid

Plasmid pSXneo-1.6-T₂AG₃ (Hanish et al., 1994), (Figure 3) was partially digested with Kpn1 restriction enzyme and products electrophoresed on a 0.8% Tris-acetate/EDTA (TAE) agarose gel. The linearised 5.2 kb band was excised from the gel and purified using Bresa-clean (Bresa-Tec). The ends were polished using 1 unit of Klenow enzyme (Boehringer Mannheim) and 50 µM dNTPs, then phenol extracted. The linearised and polished plasmid was re-ligated to itself using 5 units of T4 ligase (Boehringer Mannheim). Either Kpn1 restriction site could be destroyed using this method. Site 2 was intended to be destroyed (See Figure 3). This would allow the plasmid to be linearised with Kpn1 at site 1 which could then be used as a targeting vector and become integrated at the telomeres. The re-ligated plasmid was electroporated into cells and colonies were mini-prepped. The plasmid was again cut with Kpn1. If site 1 was destroyed, the resulting products would be 1.6 kb (consisting of telomere repeats) and 3.6 kb of plasmid. If site 2 was destroyed, the resulting products would be 0.8 kb (1 stretch of telomere repeats) and 4.4 kb of plasmid. Two clones gave products indicating site 2 had been destroyed. These were maxi-prepped (Qiagen) and used for the transfections. The modified plasmid was renamed Tel.

### Cells

GM847DM are SV40-immortalised human skin fibroblasts ("double mutant", ouabain and 6-thioguanine resistant universal hybridiser cells) obtained from Dr O Pereira-Smith, Baylor College of Medicine, Houston, Texas.

HT1080, a fibrosarcoma cell line, was obtained from the American Type Culture Collection.

A9, a mouse fibrosarcoma cell line, was obtained from the American Type Culture Collection.

### Cell Culture

All cell lines and resulting hybrids were maintained in Dulbecco's Modified Eagle Medium (DMEM) plus ~10% fetal bovine serum (FBS). Transfected GM847DM and HT1080 cells were maintained in 300 µg/ml and 700 µg/ml G418, respectively. Cell cultures were routinely maintained in 75 cm² flasks and subcultured by trypsinisation at a ratio of 1:8 or 1:16.

### Transfection

Cells, 1x10⁸, were plated in a 10 cm dish 18h prior to transfection. For transfections, 10 µg of Tel linearised with either Kpn1 (Tel) or Not1 (to create Subtel) and 100µl of Lipofectamine (GIBCO BRL) were added to the cells in a total volume of 8 ml of serum-free DME medium. Five hours after transfection, the cells were supplemented with 20% FBS. Two days after transfection cells were trypsinised and 1x10⁵ cells seeded out for selection. Eighteen hours later G418 at the appropriate concentration was added. Two weeks later when controls had died, individual foci arising after transfection were harvested by trypsinisation using plastic cloning cylinders and subcultured to give rise to clonal cell lines.

### Telomere Length Analysis

Genomic DNA was extracted from cells using a DNA extraction kit (Stratagene). Twenty micrograms of DNA was digested with Hinf1 and Rsa1 (Boehringer Mannheim), extracted once with phenol/chloroform, ethanol precipitated and resuspended in Tris-EDTA. The digested DNA was quantitated by fluorometry and 1.0 µg was electrophoresed through a 0.8% agarose gel in (TAE) buffer at 2 V cm⁻¹ for 17 h. The gel was dried for 40 min at 40°C, denatured for 30-60 min in 0.5 M NaOH and 1.5 M NaCl and neutralised for 30-60 min in 1 M Tris-Cl, pH 8.0 and 1.5 M NaCl. The gel was then hybridised to a [γ-³²P] dATP 5' end-labelled telomeric oligonucleotide probe [γ-³²P-(TTAGGG)₃]. Hybridisation and washing were carried out as previously described (Counter et al., 1992). The gel was autoradiographed on Kodak XAR5 X-ray film for 12-24 h at -80°C.

### Southern Analysis

Approximately twenty micrograms of genomic DNA was digested with Xba1 for 16 hours. The digested DNA was quantitated by fluorometry and 3.0 µg was electrophoresed through a 0.8% agarose gel in TAE buffer for 16 hours at 30V. The gel was stained in 10 pg/ml of ethidium bromide, de-purinated in 0.25 M HCl, denatured for 30 min in 0.5M NaOH and 1.5 M NaCl and neutralised for 30 min in 1 M Tris-Cl, pH 8.0 and 1.5 M NaCl, then transferred by capillary action to Hybond N+ (Amersham) membrane in 0.4 M NaOH. The membrane was hybridised to [γ⁻³²P] dCTP -pSXneo random primed using Giga-prime kit (Bresatec) at 65°C for 16 hours in a solution consisting of 5x Denhardt's (50x is 5 g Ficoll, 5 g polyvinylpyrrolidone, 5 g bovine serum albumin, H₂O to 50 ml), 6xSSC (sodium citrate, NaCl), 0.5% SDS and 50 pg/ml of denatured Herring sperm DNA. The membrane was washed 2x with 2xSSC/0.5% SDS for 10 min at 65°C, 1x with 1xSSC/0.5% SDS for 10 min at 65°C, then autoradiographed on Kodak XAR-5 or MS X-ray film for 3 days at-80°C.

### Bal-31 Analysis of DNA

Genomic DNA (100 µg) was incubated at 30°C with the exonuclease Bal-31 (10 units; Genesearch) in 600 mM NaCl, 20 mM Tris-HCl, pH 8.0, 12 mM CaCl₂, 12 mM MgCl₂,1 mM EDTA, in a total volume of 1 ml. Aliquots (100 *µ*l) were removed at 0, 0.25, 0.5, 1 and 2 hours and EDTA (40 mM) was added to stop the reaction. The DNA was ethanol precipitated, then digested with either Hinf1/Rsa1 or Sca1 and subjected to TRF analysis or Southern as described above.

### Fluorescence In Situ Hybridisation

Metaphase chromosomes were obtained according to standard cytogenetic methods for exponentially growing fibroblast cell lines.

Plasmid pSXneo was labelled with bio-16-dUTP using the Biotin-Nick Translation Mix (Boehringer Mannheim) according to the manufacturer's instructions.

Chromosome preparations were RNase A treated (200 µg/ml 2xSSC; Boehringer Mannheim) at 37°C for 60 min, rinsed in 2xSSC, briefly equilibrated in 10 mM HCl (pH 2. 0) and digested with pepsin (0. 01%/10 mM HCl; Boehringer Mannheim) at 37°C for 10 min, followed by three washes with PBS (Mg²⁺, Ca²⁺)and post-fixation in 1 % formaldehyde at RT.

Approximately 30 ng/µl of pSXneo probe was hybridised onto separately denatured chromosome preparations for 16-18 hours in a humidified chamber at 37°C. After two brief post-hybridisation washes in 50% formamide/2xSSC at 42°C and 2xSSC at RT, slides were blocked in BSA (5%/4xSSC/0.2% Tween20, Boehringer Mannheim) at RT for 10-30 min.

Detection of the hybridised pSXneo probe was performed with fluorescein-conjugated avidin DCS (5 µg/ml 4xSSC/0.2% Tween20; Vector Laboratories) or Oregon Green 519-conjugated NeutraLite avidin (5 µg/ml, 4xSSC/0.2% Tween20; Molecular Probes) followed by two amplification steps with biotinylated anti-avidin antibody (5 µg/ml, 4xSSC/0.2% Tween20; Vector Laboratories) and a second and third layer of fluorochrome-conjugated avidin.

Chromosomes were counterstained with propidium iodide (PI, 120 ng/ml final concentration; Sigma) and diamidino-phenyl-indole-dihydrochloride (DAPI, 0.6 µg/ml final concentration; Sigma) for chromosome identification, and slides were evaluated on a Leica DMLB epifluorescence microscope with appropriate filter sets for UV and blue excitation.

Between 50 and 100 metaphases were analysed for each clone, and chromosomes were scored as positive if a signal doublet could be detected on both sister chromatids of the respective chromosome.

FTTC-, PI- and DAPI-images were captured separately with a cooled CCD (SPOT2, Diagnostic Instruments) camera, merged using SPOT2 software, and further processed using Adobe Photoshop Version 5.5 software.

### Microcell-mediated Chromosome Transfer.

Microcell-mediated chromosome transfer was performed using GM847/Tel-1 as the donor and A9 as the recipient to yield the CMC3c2 hybrid line, and then CMC3c2 as the donor with GM847 cells as the recipient. Briefly, the donor cells were grown in tissue culture flasks for 48h in 20 ng/ml colcemid (Sigma), treated with 10 µg/ml cytochalasin B (Sigma) and pelleted in a Beckman J2-21 centrifuge. The pellet was then fractionated through a series of Nucleopore™ filters (Whatman) and the resulting filtrate was overlaid onto the recipient cells. The recipient cells were treated with 50% polyethylene glycol 1300-1600 (Sigma), washed and left to recover. After 24h the recipient cells were plated in 10cm dishes and selected for G418 resistance. Colonies were isolated and analyzed by FISH to determine positive microcell hybrids.

### RESULTS

### Construction of Tel Plasmid

In order to investigate the mechanism of ALT a targeting vector was required. The targeting vector needed to be targeted to the telomeres of ALT cells, then followed by FISH to see if it is copied to other telomeres which would indicate that recombination of the telomeres is occurring. The targeting vector consisted of a backbone containing the neomycin resistance gene flanked at both ends by telomere repeats. Figure 3 shows the map of pSXneo-1.6T₂AG₃ which contained two multiple cloning sites shown as site 1 and 2. The vector contained two 800bp-telomere repeats separated by cloning site 1. Both cloning sites contained the restriction sites, EcoR1, Sac1 and Kpn1. For pSXneo-1.6-T₂AG₃ to be used as a targeting vector, it needed to be linearised at cloning site 1 which would result in the backbone being flanked by telomere sequences. The Kpn1 restriction site at site 2 was destroyed as described in Materials and Methods set out above to create Tel. This plasmid was then linearised with Kpn1 at site 1 only and transfected into GM847 which are ALT cells. As a control, Tel linearised with Kpn1 was also transfected into HT1080 telomerase positive cells. As a further control, Tel was linearised with Not1 to create Subtel and transfected into GM847 and HT1080 cells. This vector would not function as a targeting vector but should still be integrated close to the telomeres. The integrated Subtel plasmid should not be copied onto other telomeres, as it would only contain telomere repeats distal to the plasmid.

G418-resistant colonies were picked and expanded. Two clones from each transfection were chosen for further study. From the Tel transfection GM847/Tel-1, GM847/Tel-2, HT1080/Tel-1 and HT1080/Tel-2 and from the Subtel transfection, GM847/Subtel-1, and GM847/Subtel-2. These clones were analysed at early and late passage by FISH using pSXneo (lacking the telomere repeats) as a probe.

### Fluorescence In Situ Hybridisation

Inter-telomeric templating was assayed by placing a plasmid tag within the telomeres of ALT cells (Figure 6). If the plasmid DNA tag is located within the telomere, inter-telomeric recombination that occurs proximal (centromeric) to the tag may result in copying of the tag to another telomere (Figure 6a). By contrast, if the tag is immediately proximal to the telomeric DNA, recombination can only occur distal to the tag which will not therefore be copied (Figure 6b).

The telomeres of ALT cells (GM847) were targeted with Tel (Figure 6a); telomeric integration was determined as described in Methods. G418-selected clones GM847/trel-1 and Tel-2 contained three and two telomeric tags, respectively, at population doubling (PD) 23, as detected by FISH. By PD63 the number of tagged telomeres within the cell population had increased to ten in both clones (Figure 7 and Table 1); the maximum number of detectable tags per metaphase was five. These data are consistent with telomere length being dynamic in ALT cells, with telomere shortening deleting some of the tags and telomere lengthening via telomere-telomere recombination resulting in copying of the tag on to previously untagged telomeres.

Subtelomeric tags were obtained by transfecting cells with a plasmid (Subtel; Figure 6b) that causes chromosomal truncation and seeding of a new telomere in both telomerase-positive and ALT cell lines. As predicted by the recombination/copy switching model (Figure 6), the number of tagged telomeres in GM847/Subtel clones did not vary with increasing PD. In GM847/Subtel-1, the same two telomeres were tagged, and in GM847/Subtel-2 only one chromosome was tagged, at both early and late PD (Figure 7 and Table 1). FISH analysis of several GM847 clones using a panel of subtelomeric probes did not detect subtelomeric translocation events, further demonstrating that the recombination events specifically involve the telomeres. To test whether the effect was specific for ALT, the Tel plasmid was transfected into telomerase-positive fibrosarcoma HT1080 cells. HT1080/Tel-1 and /Tel-2 showed no increase in the number of tagged telomeres from early to later PD level (Figure 7 and Table 1).

The FISH data were confirmed by Southern analysis of integrated plasmid DNA: the GM847/Tel-1 and Tel-2 lines exhibited an increase in the number of hybridising bands from early to later PD level (Figure 8). This was most recognised for clone GM847/Tel-2 where PD63 cells had seven bands not present at PD23. In contrast, the GM847/Subtel-1 and Subtel-2 lines and the HT1080/Tel-1 and Tel-2 lines contained far fewer bands, and the number of bands did not change (Figure 8). The bands present in the GM847/Tel clones differed in relative intensity from early to late PD, suggesting loss of plasmid tags from some telomeres and enrichment of the population with cells containing other tags.

To demonstrate definitively that the tag from a single telomere was copied into other telomeres, a single tagged chromosome from GM847/Tel-1 cells was transferred into the mouse A9 cell line using microcell-mediated chromosome transfer (MMCT) followed by G418 selection, creating the hybrid cell line CMC3c2 (Figure 9a). Four bands hybridising to the plasmid core were detected by Southern analysis, and as expected each one corresponded to a band present in the GM847/Tel-1 cells (Figure 8). The tagged chromosome was then transferred by MMCT from CMC3c2 into GM847 cells. At PD 24 the tag was already detected at more than one telomere (Figure 9b).

These data demonstrate for the first time that in ALT cells telomeric DNA is copied to other telomeres. The proteins involved in this process need to be identified, but it is highly likely that they are proteins involved in DNA recombination and replication rather than proteins involved only in telomere maintenance. Telomere-telomere recombination was not detected in the telomerase-positive HT1080 cell line. This is consistent with the finding that inhibition of telomerase in HT1080 cells results in telomere shortening and the onset of a senescence-like state, indicating that these cells do not possess a telomere maintenance mechanism other than telomerase.

**Table 1: Telomere-tagged chromosomes in GM847 and HT1080 clones**

| Cell line | Early^{a} PD | Late^{a} PD |
|---|---|---|
| GM847/trel-1 | 10pter, 13pter, 16pter | 5pter, 10pter, 13pter, 16pter, 17qter. 18pter, 20pter, 21pter, Cpter, Cqter |
| GM847/Trel-2 | 16pter, small marker | 1pter, 1qter, 2pter, 7pter, 3pter, 16pter, small marker, Bqter, Cpter, marker |
| GM847/Subtel-1 | 1pter, small marker | 1pter, small marker |
| GM847/Subtel-2 | 13qter | 13qter |
| HT1080/Tel-1 | 2pter | 2pter |
| HT1080/Tel-2 | 15pter | 15pter |

| | | |
|---|---|---|
| ^{a}Early and late PD levels are specified in legend to Figure 7. | | |

### References:

Colgin, L. M. and Reddel, R R. (1999). *Curr. Opin. Genet. Dev.* **9,** 97-103. Bryan, T. M., Englezou, A., Gupta, J., Bacchetti, S., and Reddel, R. R. (1995).
*EMBO J.* **14,** 4240-4248.
Yeager, T. R., Neumann, A. A., Englezou, A., Huschtscha, L. I., Noble, J. R., and Reddel, R. R. (1999). *Cancer Res.* **59**, 4175-4179.
Reddel, R. R., Bryan, T. M., and Murnane, J. P. (1997). *Biochemistry (Mosc)* **62***,* 1254-1262.
Reddel, R. R (2000). *Carcinogenesis* **21**: 477-484.
Hanish, J. P., Yanowitz, J. L. and de Lange (1994). Proc. Natl. Acad. Sci. USA, 91:8861-8865.
Counter et al, (1992). EMBO J 11:1921-1929.

## Claims

1. A method of assaying for ALT in a eukaryotic cell, which method comprises:
(a) introducing into the cell a nucleic acid construct capable of integrating a DNA tag sequence into a telomere by homologous recombination;
(b) selecting cells having the DNA tag sequence integrated into one or more telomeres;
(c) allowing cells from (b) to undergo division; and
(d) determining the presence of the DNA tag sequence in additional telomeres,
wherein the nucleic acid construct comprises the DNA tag sequence linked to (i) a first DNA sequence positioned 3' of the DNA tag sequence and (ii) a second DNA sequence positioned 5' of the DNA tag sequence, the said first and second DNA sequences each comprising multiple repeats homologous to eukaryotic telomere DNA, and the DNA tag sequence comprising a marker.

2. The method according to claim 1, wherein the multiple repeats are (17AGGG)n.

3. The method according to claim 1 or claim 2, wherein the DNA tag sequence further comprises a prokaryotic selectable marker positioned 5' to the marker and the construct comprises a endonuclease recognition site positioned 3' of the prokaryotic selectable marker.

4. An assay method for screening a compound for an effect on ALT activity in a cell, the method comprising:
(a) providing a cell having ALT activity and comprising a DNA tag sequence comprising a marker, the DNA tag sequence being integrated into one or more telomeres of said cell;
(b) contacting the cell with a test compound;
(c) allowing the cell to undergo division; and
(d) determining in the progeny of the cell whether there is any change in the rate or incidence of telomeric incorporation of the DNA tag sequence in additional telomeres as compared with untreated cells.

5. An assay method for screening a compound for an effect on ALT activity in a cell, the method comprising:
(a) providing a cell having ALT activity and comprising (i) a first DNA tag sequence comprising a first marker, the first DNA tag sequence being integrated into a telomere of a first chromosome of said cell; and (ii) a second DNA tag sequence comprising a second marker, the second DNA tag sequence being integrated into a second chromosome of said cell at a subtelomeric position;
(b) contacting the cell with a test compound;
(c) allowing the cell to undergo division; and
(d) determining in the progeny of the cell whether there is any change in the rate or incidence with which the first DNA tag sequence is copied into a telomere of a chromosome that contains the second DNA tag sequence as compared with an untreated cell.

6. The method according to claim 5, wherein
the first DNA tag sequence further comprises a first prokaryotic selectable marker positioned 5' to the first marker and a first endonuclease recognition site positioned 3' of the first prokaryotic selectable marker,
the second DNA tag sequence further comprises a bacterial origin of replication, a second prokaryotic selectable marker positioned 3' to the second marker and a second endonuclease recognition site positioned 5' of the second prokaryotic selectable marker,
and step (d) comprises
(i) recovering nucleic acids from the cell;
(ii) contacting the recovered nucleic acids with one or more endonucleases which cleave the first and second endonuclease recognition sites in both the first and second DNA tag sequences;
(iii) contacting the nucleic acids from step (ii) with an enzyme that catalyses intramolecular ligation of the nucleic acids;
(iv) introducing the nucleic acids from step (iii) into one or more bacterial cells; and
(v) selecting bacterial cells that comprise the first prokaryotic selectable marker and the second prokaryotic selectable marker.

7. The method according to claim 6, wherein the cell having ALT activity comprises a chromosome having a third DNA tag sequence integrated at an interstitial site, the third DNA tag sequence comprising a third prokaryotic selectable marker, the third DNA tag sequence is flanked by endonuclease recognition sites, step (ii) further comprises contacting the recovered nucleic acids with one or more endonucleases that cleave the endonuclease recognition sites flanking the third DNA tag sequence; and step (v) further comprises selecting bacterial cells that comprise the third prokaryotic selectable marker.

8. The method according to claim 6 or claim 7, wherein the telomeric sequences positioned 3' to the second DNA sequence tag integrated at a subtelomeric position comprise a third unique endonuclease recognition site and the method further comprises, prior to step (b), a step of introducing into the cell a third endonuclease which cleaves the third unique endonuclease recognition site in said telomeric sequences.

9. Use of a method according to any one of claims 1 to 8 to identify anti-cancer compounds.

10. An assay method for determining whether a gene product affects ALT activity in a eukaryotic cell, the method comprising:
(a) providing a cell having ALT activity and comprising a DNA tag sequence comprising a marker, the DNA tag sequence being integrated into one or more telomeres of said cell;
(b) altering the levels of the gene product in said cell;
(c) allowing the cell to undergo division; and
(d) determining in the progeny of the cell whether there is any change in the rate or incidence of telomeric incorporation of the DNA tag sequence in additional telomeres as compared with control cells.

11. An assay method for determining whether a gene product affects ALT activity in a eukaryotic cell, the method comprising:
(a) providing a cell having ALT activity and comprising (i) a first DNA tag sequence comprising a first marker, the first DNA tag sequence being integrated into a telomere of a first chromosome of said cell; (ii) a second DNA tag sequence comprising a second marker, the second DNA tag sequence being integrated into a second chromosome of said cell at a subtelomeric position;
(b) altering the levels of the gene product in said cell;
(c) allowing the cell to undergo division; and
(d) determining in the progeny of the cell whether there is any change in the rate or incidence with which the first DNA tag sequence is copied into a telomere of a chromosome that contains the second DNA tag sequence as compared with control cells.

12. The method according to claim 11 wherein the cell having ALT activity comprises a third chromosome that comprises a third DNA tag sequence integrated at an interstitial site, the third DNA tag sequence comprising a third marker.

13. The method according to claim 11, wherein
the first DNA tag sequence further comprises a first prokaryotic selectable marker positioned 5' to the first marker and a first endonuclease recognition site positioned 3' of the first prokaryotic selectable marker,
the second DNA tag sequence further comprises a bacterial origin of replication, a second prokaryotic selectable marker positioned 3' to the second marker and a second endonuclease recognition site positioned 5' of the second prokaryotic selectable marker,
and step (d) comprises
(i) recovering nucleic acids from the cell;
(ii) contacting the recovered nucleic acids with one or more endonucleases which cleave the first and second endonuclease recognition sites in both the first and second DNA tag sequences;
(iii) contacting the nucleic acids from step (ii) with an enzyme that catalyses intramolecular ligation of the nucleic acids;
(iv) introducing the nucleic acids from step (iii) into one or more bacterial cells; and
(v) selecting bacterial cells that comprise the first prokaryotic selectable marker and the second prokaryotic selectable marker.

14. The method according to claim 13, wherein the cell having ALT activity comprises a chromosome having a third DNA tag sequence integrated at an interstitial site, the third DNA tag sequence comprising a third prokaryotic selectable marker, the third DNA tag sequence is flanked by endonuclease recognition sites, step (ii) further comprises contacting the recovered nucleic acids with one or more endonucleases that cleave the endonuclease recognition sites flanking the third DNA tag sequence; and step (v) further comprises selecting bacterial cells that comprise the third prokaryotic selectable marker.

15. The method according to claim 13 or claim 14, wherein the telomeric sequences positioned 3' to the second DNA sequence tag integrated at a subtelomeric position comprise a third unique endonuclease recognition site and the method further comprises, prior to step (b), a step of introducing into the cell a third endonuclease which cleaves the third unique endonuclease recognition site in said telomeric sequences.

16. The method according to any one of claims 10 to 15, wherein the levels of the gene product are altered by introducing into said cell a nucleic acid which is capable of directing expression of the gene product in said cell and incubating the cell under conditions that cause expression of the gene product.

17. Use of a nucleic acid construct in a method for assaying for ALT activity in eukaryotic cells, wherein the nucleic acid construct is capable of integrating a DNA tag sequence into a telomere by homologous recombination, the construct comprising the DNA tag sequence linked to (i) a first DNA sequence positioned 3' of the tag sequence and (ii) a second DNA sequence positioned 5' of the first DNA tag sequence, the said first and second DNA sequences each comprising multiple repeats homologous to eukaryotic telomere DNA, and the DNA tag sequence comprising a first marker.

18. The use according to claim 17, wherein the multiple repeats are (TTAGGG)n.

19. The use according to claim 17 or claim 18, wherein the DNA tag sequence further comprises a first prokaryotic selectable marker positioned 5' to the first marker and the construct comprises a first endonuclease recognition site positioned 3' of the first prokaryotic selectable marker.

20. Use of a nucleic acid construct in a method for assaying for ALT activity in eukaryotic cells, wherein the nucleic acid construct is capable of integrating a DNA tag sequence into a subtelomeric position within a chromosome, the construct comprising the DNA tag sequence linked to (i) a first DNA sequence positioned 3' of the DNA tag sequence, which first DNA sequence comprises multiple repeats homologous to eukaryotic telomere DNA, and optionally (ii) a third DNA sequence positioned 5' of the DNA tag sequence, wherein any DNA sequence 5' of the DNA tag sequence does not contain a nucleic acid sequence homologous to eukaryotic telomere DNA, and the DNA tag sequence comprises a second marker

21. The use according to claim 20, wherein the multiple repeats are (TTAGGG)n.

22. The use according to claim 20 or claim 21, wherein the DNA tag sequence further comprises a bacterial origin of replication, a second prokaryotic selectable marker positioned 3' to the second marker and the construct comprises a second endonuclease recognition site positioned 5' of the second prokaryotic selectable marker.

## Patentansprüche

1. Verfahren zum Testen hinsichtlich ALT in einer eukaryontischen Zelle, wobei das Verfahren folgendes umfaßt:
(a) Einbringen eines Nukleinsäurekonstrukts, das in der Lage ist, mittels homologer Rekombination eine DNA-Markierungssequenz in ein Telomer zu integrieren, in die Zelle;
(b) Selektieren von Zellen, welche die erste DNA-Markierungssequenz in ein oder mehrere Telomere integriert haben;
(c) Zulassen, daß die Zellen aus (b) sich teilen; und
(d) Feststellen des Vorhandenseins der ersten DNA-Markierungssequenz in weiteren Telomeren,
wobei das Nukleinsäurekonstrukt die DNA-Markierungssequenz in Verknüpfung mit (i) einer ersten DNA-Sequenz, die 3' zu der DNA-Markierungssequenz angeordnet ist, und (ii) einer zweiten DNA-Sequenz, die 5' zu der DNA-Markierungssequenz angeordnet ist, umfaßt, wobei die erste und die zweite DNA-Sequenz jeweils mehrere Wiederholungen umfassen, die zu eukaryontischer telomerer DNA homolog sind, und wobei die DNA-Markierungssequenz einen Marker umfaßt.

2. Verfahren nach Anspruch 1, wobei die mehreren Wiederholungen (TTAGGG)n sind.

3. Verfahren nach Anspruch 1 oder Anspruch 2, wobei die DNA-Markierungssequenz weiterhin einen prokaryontischen selektierbaren Marker, der 5' zu dem Marker angeordnet ist, umfaßt und das Konstrukt eine Endonukleaseerkennungsstelle, die 3' zu dem prokaryontischen selektierbaren Marker angeordnet ist, umfaßt.

4. Testverfahren zur Durchmusterung einer Verbindung hinsichtlich einer Wirkung auf ALT-Aktivität in einer Zelle, wobei das Verfahren folgendes umfaßt:
(a) Bereitstellen einer Zelle, die ALT-Aktivität aufweist und eine DNA-Markierungssequenz, die einen Marker aufweist, umfaßt, wobei die DNA-Markierungssequenz in ein oder mehrere Telomere der Zelle integriert ist;
(b) Inkontaktbringen der Zelle mit einer Testverbindung;
(c) Zulassen, daß die Zelle sich teilt; und
(d) Feststellen in den Nachkommen der Zelle, ob es eine Veränderung in der Geschwindigkeit oder dem Auftreten von Telomeraufnahme der DNA-Markierungssequenz in weiteren Telomeren gibt im Vergleich zu unbehandelten Zellen.

5. Testverfahren zur Durchmusterung einer Verbindung hinsichtlich einer Wirkung auf ALT-Aktivität in einer Zelle, wobei das Verfahren folgendes umfaßt:
(a) Bereitstellen einer Zelle, die ALT-Aktivität aufweist und (i) eine erste DNA-Markierungssequenz, die einen ersten Marker aufweist, umfaßt, wobei die erste DNA-Markierungssequenz in ein Telomer eines ersten Chromosoms der Zelle integriert ist, und (ii) eine zweite DNA-Markierungssequenz, die einen zweiten Marker aufweist, umfaßt, wobei die zweite DNA-Markierungssequenz in ein zweites Chromosom der Zelle in einer subtelomeren Position integriert ist;
(b) Inkontaktbringen der Zelle mit einer Testverbindung;
(c) Zulassen, daß die Zelle sich teilt; und
(d) Feststellen in den Nachkommen der Zelle, ob es eine Veränderung in der Geschwindigkeit oder dem Auftreten, mit welchem die erste DNA-Markierungssequenz in ein Telomer eines Chromosoms, das die zweite DNA-Markierungssequenz enthält, kopiert wird, im Vergleich zu einer unbehandelten Zelle.

6. Verfahren nach Anspruch 5, wobei
die erste DNA-Markierungssequenz weiterhin einen ersten prokaryontischen selektierbaren Marker, der 5' zu dem ersten Marker angeordnet ist, und eine erste Endonukleaseerkennungsstelle, die 3' zu dem ersten prokaryontischen selektierbaren Marker angeordnet ist, umfaßt,
die zweite DNA-Markierungssequenz weiterhin einen bakteriellen Replikationsursprung, einen zweiten prokaryontischen selektierbaren Marker, der 3' zu dem zweiten Marker angeordnet ist, und eine zweite Endonukleaseerkennungsstelle, die 5' zu dem zweiten prokaryontischen selektierbaren Marker angeordnet ist, umfaßt,
und Stufe (d) folgendes umfaßt:
(i) Gewinnen von Nukleinsäuren aus der Zelle;
(ii) Inkontaktbringen der gewonnenen Nukleinsäuren mit einer oder mehreren Endonukleasen, welche die ersten und zweiten Endonukleaseerkennungsstellen in sowohl der ersten als auch der zweiten DNA-Markierungssequenz spalten;
(iii) Inkontaktbringen der Nukleinsäuren aus Stufe (ii) mit einem Enzym, das eine intramolekulare Ligation der Nukleinsäuren katalysiert;
(iv) Einbringen der Nukleinsäuren aus Stufe (iii) in eine oder mehrere Bakterienzellen und
(v) Selektieren von Bakterienzellen, welche den ersten prokaryontischen selektierbaren Marker und den zweiten prokaryontischen selektierbaren Marker enthalten.

7. Verfahren nach Anspruch 6, wobei die Zelle mit ALT-Aktivität ein Chromosom mit einer dritten DNA-Markierungssequenz, die an einer Zwischenstelle integriert ist, umfaßt, wobei die dritte DNA-Markierungssequenz einen dritten prokaryontischen selektierbaren Marker umfaßt, die dritte DNA-Markierungssequenz von Endonukleaseerkennungsstellen flankiert ist, Stufe (ii) weiterhin umfaßt, daß man die gewonnenen Nukleinsäuren mit einer oder mehreren Endonukleasen in Kontakt bringt, welche die Endonukleaseerkennungsstellen, welche die dritte DNA-Markierungssequenz flankieren, spalten, und Stufe (v) weiterhin umfaßt, daß man Bakterienzellen selektiert, welche den dritten prokaryontischen selektierbaren Marker umfassen.

8. Verfahren nach Anspruch 6 oder Anspruch 7, wobei die Telomersequenzen, die 3' zu der zweiten DNA-Sequenzmarkierung, die an einer subtelomeren Position integriert ist, angeordnet sind, eine dritte einzigartige Endonukleaseerkennungsstelle umfassen, und wobei das Verfahren vor der Stufe (b) weiterhin eine Stufe umfaßt, bei der man eine dritte Endonuklease in die Zelle einbringt, welche die dritte einzigartige Endonukleaseerkennungsstelle in den Telomersequenzen spaltet.

9. Verwendung eines Verfahrens nach einem der Ansprüche 1 bis 8 zur Identifizierung von Anti-Krebs-Verbindungen.

10. Testverfahren zum Feststellen, ob ein Genprodukt ALT-Aktivität in einer eukaryontischen Zelle beeinflußt, wobei das Verfahren folgendes umfaßt:
(a) Bereitstellen einer Zelle, die ALT-Aktivität aufweist und eine DNA-Markierungssequenz, die einen Marker aufweist, umfaßt, wobei die DNA-Markierungssequenz in ein oder mehrere Telomere der Zelle integriert ist;
(b) Veränderung der Mengen des Genprodukts in der Zelle;
(c) Zulassen, daß die Zelle sich teilt; und
(d) Feststellen in den Nachkommen der Zelle, ob es eine Veränderung in der Geschwindigkeit oder dem Auftreten von Telomeraufnahme der DNA-Markierungssequenz in weiteren Telomeren gibt im Vergleich zu Kontrollzellen.

11. Testverfahren zum Feststellen, ob ein Genprodukt ALT-Aktivität in einer eukaryontischen Zelle beeinflußt, wobei das Verfahren folgendes umfaßt:
(a) Bereitstellen einer Zelle, die ALT-Aktivität aufweist und (i) eine erste DNA-Markierungssequenz, die einen ersten Marker aufweist, umfaßt, wobei die erste DNA-Markierungssequenz in ein Telomer eines ersten Chromosoms der Zelle integriert ist, (ii) eine zweite DNA-Markierungssequenz, die einen zweiten Marker aufweist, umfaßt, wobei die zweite DNA-Markierungssequenz in ein zweites Chromosom der Zelle an einer subtelomeren Position integriert ist;
(b) Verändern der Mengen des Genprodukts in der Zelle;
(c) Zulassen, daß die Zelle sich teilt; und
(d) Feststellen in den Nachkommen der Zelle, ob es eine Veränderung in der Geschwindigkeit oder dem Auftreten, mit welchem die erste DNA-Markierungssequenz in ein Telomer eines Chromosoms, das die zweite DNA-Markierungssequenz enthält, kopiert wird, gibt, im Vergleich zu Kontrollzellen.

12. Verfahren nach Anspruch 11, wobei die Zelle, die ALT-Aktivität aufweist, ein drittes Chromosom umfaßt, das eine dritte DNA-Markierungssequenz enthält, die an einer Zwischenstelle integriert ist, wobei die dritte DNA-Markierungssequenz einen dritten Marker umfaßt.

13. Verfahren nach Anspruch 11, wobei
die erste DNA-Markierungssequenz weiterhin einen ersten prokaryontischen selektierbaren Marker, der 5' zu dem ersten Marker angeordnet ist, und eine erste Endonukleaseerkennungsstelle, die 3' zu dem ersten prokaryontischen selektierbaren Marker angeordnet ist, umfaßt,
die zweite DNA-Markierungssequenz weiterhin einen bakteriellen Replikationsursprung, einen zweiten prokaryontischen selektierbaren Marker, der 3' zu dem zweiten Marker angeordnet ist, und eine zweite Endonukleaseerkennungsstelle, die 5' zu dem zweiten prokaryontischen selektierbaren Marker angeordnet ist, umfaßt,
und Stufe (d) folgendes umfaßt:
(i) Gewinnen von Nukleinsäuren aus der Zelle;
(ii) Inkontaktbringen der gewonnenen Nukleinsäuren mit einer oder mehreren Endonukleasen, welche die ersten und zweiten Endonukleaseerkennungsstellen in sowohl der ersten als auch der zweiten DNA-Markierungssequenz spalten;
(iii) Inkontaktbringen der Nukleinsäuren aus Stufe (ii) mit einem Enzym, das eine intramolekulare Ligation der Nukleinsäuren katalysiert;
(iv) Einbringen der Nukleinsäuren aus Stufe (iii) in eine oder mehrere Bakterienzellen und
(v) Selektieren von Bakterienzellen, welche den ersten prokaryontischen selektierbaren Marker und den zweiten prokaryontischen selektierbaren Marker enthalten.

14. Verfahren nach Anspruch 13, wobei die Zelle mit ALT-Aktivität ein Chromosom mit einer dritten DNA-Markierungssequenz, die an einer Zwischenstelle integriert ist, umfaßt, wobei die dritte DNA-Markierungssequenz einen dritten prokaryontischen selektierbaren Marker umfaßt, die dritte DNA-Markierungssequenz von Endonukleaseerkennungsstellen flankiert ist, Stufe (ii) weiterhin umfaßt, daß man die gewonnenen Nukleinsäuren mit einer oder mehreren Endonukleasen in Kontakt bringt, welche die Endonukleaseerkennungsstellen, welche die dritte DNA-Markierungssequenz flankieren, spalten, und Stufe (v) weiterhin umfaßt, daß man Bakterienzellen selektiert, welche den dritten prokaryontischen selektierbaren Marker umfassen.

15. Verfahren nach Anspruch 13 oder Anspruch 14, wobei die Telomersequenzen, die 3' zu der zweiten DNA-Sequenzmarkierung, die an einer subtelomeren Position integriert ist, angeordnet sind, eine dritte einzigartige Endonukleaseerkennungsstelle umfassen, und wobei das Verfahren vor der Stufe (b) weiterhin eine Stufe umfaßt, bei der man eine dritte Endonuklease in die Zelle einbringt, welche die dritte einzigartige Endonukleaseerkennungsstelle in den Telomersequenzen spaltet.

16. Verfahren nach einem der Ansprüche 10 bis 15, wobei die Mengen des Genprodukts verändert werden, indem man eine Nukleinsäure in die Zelle einbringt, welche in der Lage ist, die Expression des Genprodukts in der Zelle zu steuern, und man die Zelle unter Bedingungen inkubiert, die eine Expression des Genprodukts bewirken.

17. Verwendung eines Nukleinsäurekonstrukts in einem Verfahren zum Testen hinsichtlich ALT-Aktivität in eukaryontischen Zellen, wobei das Nukleinsäurekonstrukt in der Lage ist, mittels homologer Rekombination eine DNA-Markierungssequenz in ein Telomer zu integrieren, wobei das Konstrukt die DNA-Markierungssequenz umfaßt, die mit (i) einer ersten DNA-Sequenz, die 3' zu der Markierungssequenz angeordnet ist, und (ii) einer zweiten DNA-Sequenz, die 5' zu der ersten DNA-Markierungssequenz angeordnet ist, verknüpft ist, wobei die erste und die zweite DNA-Sequenz jeweils mehrere Wiederholungen umfassen, die zu eukaryontischer telomerer DNA homolog sind, und wobei die DNA-Markierungssequenz einen ersten Marker umfaßt.

18. Verwendung nach Anspruch 17, wobei die mehreren Wiederholungen (TTAGGG)n sind.

19. Verwendung nach Anspruch 17 oder Anspruch 18, wobei die DNA-Markierungssequenz weiterhin einen ersten prokaryontischen selektierbaren Marker, der 5' zu dem ersten Marker angeordnet ist, umfaßt und das Konstrukt eine erste Endonukleaseerkennungsstelle, die 3' zu dem ersten prokaryontischen selektierbaren Marker angeordnet ist, umfaßt.

20. Verwendung eines Nukleinsäurekonstrukts in einem Verfahren zum Testen hinsichtlich ALT-Aktivität in eukaryontischen Zellen, wobei das Nukleinsäurekonstrukt in der Lage ist, eine DNA-Markierungssequenz an einer subtelomeren Position innerhalb eines Chromosoms zu integrieren, wobei das Konstrukt die DNA-Markierungssequenz umfaßt, die mit (i) einer ersten DNA-Sequenz, die 3' zu der DNA-Markierungssequenz angeordnet ist, wobei die erste DNA-Sequenz mehrere Wiederholungen, die zu eukaryontischer telomerer DNA homolog sind, umfaßt, und optional mit (ii) einer dritten DNA-Sequenz, die 5' zu der DNA-Markierungssequenz angeordnet ist, verknüpft ist, wobei jegliche DNA-Sequenz, die sich 5' zu der DNA-Markierungssequenz befindet, keine Nukleinsäuresequenz enthält, die homolog zu eukaryontischer telomerer DNA ist, und wobei die DNA-Markierungssequenz einen zweiten Marker umfaßt.

21. Verwendung nach Anspruch 20, wobei die mehreren Wiederholungen (TTAGGG)n sind.

22. Verwendung nach Anspruch 20 oder Anspruch 21, wobei die DNA-Markierungssequenz weiterhin einen bakteriellen Replikationsursprung und einen zweiten prokaryontischen selektierbaren Marker umfaßt, der 3' zu dem zweiten Marker angeordnet ist, und wobei das Konstrukt eine zweite Endonukleaseerkennungsstelle umfaßt, die 5' zu dem zweiten prokaryontischen selektierbaren Marker angeordnet ist.

## Revendications

1. Méthode d'analyse d'ALT dans une cellule eucaryotique, méthode qui comprend les étapes consistant :
(a) à introduire dans la cellule un produit d'assemblage d'acide nucléique apte à l'intégration d'une séquence de marquage d'ADN dans un télomère par recombinaison homologue ;
(b) à sélectionner les cellules comprenant la séquence de marquage d'ADN intégrée dans un ou plusieurs télomères ;
(c) à laisser les cellules de (b) subir une division ; et
(d) à déterminer la présence de la séquence de marquage d'ADN dans les télomères supplémentaires,
dans lequel le produit d'assemblage d'acide nucléique comprend la séquence de marquage d'ADN liée à (i) une première séquence d'ADN en position 3' de la séquence de marquage d'ADN et (ii) une seconde séquence d'ADN en position 5' de la séquence de marquage d'ADN, lesdites première et seconde séquences d'ADN comprenant chacune des séquences répétées multiples homologues de l'ADN télomère eucaryotique, et la séquence de marquage d'ADN comprenant un marqueur.

2. Méthode suivant la revendication 1, dans laquelle les séquences répétées multiples sont des séquences (TTAGGG)n.

3. Méthode suivant la revendication 1 ou la revendication 2, dans laquelle la séquence de marquage d'ADN comprend en outre un marqueur sélectionnable procaryotique en position 5' du marqueur et le produit d'assemblage comprend un site de reconnaissance par endonucléase en position 3' du marqueur sélectionnable procaryotique.

4. Méthode d'analyse pour sélectionner un composé pour un effet sur l'activité d'ALT dans une cellule, méthode comprenant les étapes consistant :
(a) à fournir une cellule ayant une activité d'ALT et comprenant une séquence de marquage d'ADN comprenant un marqueur, la séquence de marquage d'ADN étant intégrée à un ou plusieurs télomères de ladite cellule ;
(b) à mettre en contact la cellule avec un composé d'essai ;
(c) à laisser la cellule subir une division ; et
(d) à déterminer dans la descendance de la cellule s'il existe une quelconque variation du taux ou de l'incidence d'incorporation aux télomères de la séquence de marquage d'ADN dans des télomères supplémentaires, par comparaison avec les cellules non traitées.

5. Méthode d'analyse pour sélectionner un composé pour un effet sur l'activité d'ALT dans une cellule, méthode comprenant les étapes consistant:
(a) à fournir une cellule ayant une activité d'ALT et comprenant (i) une première séquence de marquage d'ADN comprenant un premier marqueur, la première séquence de marquage d'ADN étant intégrée à un télomère d'un premier chromosome de ladite cellule ; et (ii) une seconde séquence de marquage d'ADN comprenant un second marqueur, la seconde séquence de marquage d'ADN étant intégrée à un second chromosome de ladite cellule à une position sous-télomère ;
(b) à mettre en contact la cellule avec un composé d'essai ;
(c) à laisser la cellule subir une division ; et
(d) à déterminer dans la descendance de la cellule s'il existe une quelconque variation du taux ou de l'incidence auquel la première séquence de marquage d'ADN est copiée dans un télomère d'un chromosome qui contient la seconde séquence de marquage d'ADN, par comparaison avec une cellule non traitée.

6. Méthode suivant la revendication 5, dans laquelle
la première séquence de marquage d'ADN comprenant en outre un premier marqueur sélectionnable procaryotique en position 5' par rapport au premier marqueur et un premier site de reconnaissance par endonucléase en position 3' du premier marqueur sélectionnable procaryotique,
la seconde séquence de marquage d'ADN comprend en outre une origine bactérienne de réplication, un second marqueur sélectionnable procaryotique en position 3' par rapport à au second marqueur et un second site de reconnaissance par endonucléase en position 5' du second marqueur sélectionnable procaryotique,
et l'étape (d) comprend les étapes consistant :
(i) à recueillir les acides nucléiques à partir de la cellule ;
(ii) à mettre en contact les acides nucléiques recueillis avec une ou plusieurs endonucléases, qui clivent les premier et second sites de reconnaissance par endonucléases dans les première et seconde séquences de marquage d'ADN ;
(iii) à mettre en contact les acides nucléiques de l'étape (ii) avec une enzyme qui catalyse la ligation intramoléculaire des acides nucléiques ;
(iv) à introduire les acides nucléiques de l'étape (iii) dans une ou plusieurs cellules bactériennes ; et
(v) à sélectionner les cellules bactériennes qui comprennent le premier marqueur sélectionnable procaryotique et le second marqueur sélectionnable procaryotique.

7. Méthode suivant la revendication 6, dans laquelle la cellule ayant une activité d'ALT comprend un chromosome ayant une troisième séquence de marquage d'ADN intégrée à un site interstitiel, la troisième séquence de marquage d'ADN comprenant un troisième marqueur sélectionnable procaryotique, la troisième séquence de marquage d'ADN étant flanquée par des sites de reconnaissance par endonucléases, l'étape (ii) comprend en outre la mise en contact des acides nucléiques recueillis avec une ou plusieurs endonucléases qui clivent les sites de reconnaissance par endonucléases flanquant la troisième séquence de marquage d'ADN ; et l'étape (v) comprend en outre la sélection des cellules bactériennes qui comprennent le troisième marqueur sélectionnable procaryotique.

8. Méthode suivant la revendication 6 ou la revendication 7, dans laquelle les séquences télomères en position 3' par rapport à la seconde séquence de marquage d'ADN intégrée à une position sous-télomère comprennent un troisième site unique de reconnaissance par endonucléase et la méthode comprend en outre, avant l'étape (b), une étape d'introduction dans la cellule d'une troisième endonucléase qui clive le troisième site unique de reconnaissance par endonucléase dans lesdites séquences télomères.

9. Utilisation d'une méthode suivant l'une quelconque des revendications 1 à 8 pour identifier des composés anticancéreux.

10. Méthode d'analyse pour déterminer si un produit de gène a un effet sur l'activité d'ALT dans une cellule eucaryotique, méthode comprenant les étapes consistant:
(a) à fournir une cellule ayant une activité d'ALT et comprenant une séquence de marquage d'ADN comprenant un marqueur, la séquence de marquage d'ADN étant intégrée à un ou plusieurs télomères de ladite cellule ;
(b) à modifier les taux du produit de gène dans ladite cellule ;
(c) à laisser la cellule subir une division ; et
(d) à déterminer dans la descendance de la cellule s'il existe une quelconque variation du taux ou de l'incidence d'incorporation aux télomères de la séquence de marquage d'ADN dans des télomères supplémentaires, par comparaison avec les cellules témoins.

11. Méthode d'analyse pour déterminer si un produit de gène a un effet sur l'activité d'ALT dans une cellule eucaryotique, méthode comprenant les étapes consistant :
(a) à fournir une cellule ayant une activité d'ALT et comprenant (i) une première séquence de marquage d'ADN comprenant un premier marqueur, la première séquence de marquage d'ADN étant intégrée à un télomère d'un premier chromosome de ladite cellule ; (ii) une seconde séquence de marquage d'ADN comprenant un second marqueur, la seconde séquence de marquage d'ADN étant intégrée à un second chromosome de ladite cellule à une position sous-télomère ;
(b) à modifier les taux du produit de gène dans ladite cellule ;
(c) à laisser la cellule subir une division ; et
(d) à déterminer dans la descendance de la cellule s'il existe une quelconque variation du taux ou de l'incidence auquel la première séquence de marquage d'ADN est copiée dans un télomère d'un chromosome qui contient la seconde séquence de marquage d'ADN, par comparaison avec les cellules témoins.

12. Méthode suivant la revendication 11, dans laquelle la cellule ayant une activité d'ALT comprend un troisième chromosome qui comprend une troisième séquence de marquage d'ADN intégrée à un site interstitiel, la troisième séquence de marquage d'ADN comprenant un troisième marqueur.

13. Méthode suivant la revendication 11, dans laquelle
la première séquence de marquage d'ADN comprend en outre un premier marqueur sélectionnable procaryotique en position 5' par rapport au premier marqueur et un premier site de reconnaissance par endonucléase en position 3' du premier marqueur sélectionnable procaryotique,
la seconde séquence de marquage d'ADN comprend en outre une origine bactérienne de réplication, un second marqueur sélectionnable procaryotique en position 3' par rapport au second marqueur et un second site de reconnaissance par endonucléase en position 5' du second marqueur sélectionnable procaryotique,
et l'étape (d) comprend les étapes consistant:
(i) à recueillir les acides nucléiques à partir de la cellule ;
(ii) à mettre en contact les acides nucléiques recueillis avec une ou plusieurs endonucléases qui clivent les premier et second sites de reconnaissance par endonucléase dans les première et seconde séquences de marquage d'ADN ;
(iii) à mettre en contact les acides nucléiques de l'étape (ii) avec une enzyme qui catalyse la ligation intramoléculaire des acides nucléiques ;
(iv) à introduire les acides nucléiques de l'étape (iii) dans une ou plusieurs cellules bactériennes ; et
(v) à sélectionner les cellules bactériennes qui comprennent le premier marqueur sélectionnable procaryotique et le second marqueur sélectionnable procaryotique.

14. Méthode suivant la revendication 13, dans laquelle la cellule ayant une activité d'ALT comprend un chromosome ayant une troisième séquence de marquage d'ADN intégrée à un site interstitiel, la troisième séquence de marquage d'ADN comprenant un troisième marqueur sélectionnable procaryotique, la troisième séquence de marquage d'ADN est flanquée par des sites de reconnaissance par endonucléases, l'étape (ii) comprend en outre la mise en contact des acides nucléiques recueillis avec une ou plusieurs endonucléases qui clivent les sites de reconnaissance par endonucléases flanquant la troisième séquence de marquage d'ADN ; et l'étape (v) comprend en outre la sélection de cellules bactériennes qui comprennent le troisième marqueur sélectionnable procaryotique.

15. Méthode suivant la revendication 13 ou la revendication 14, dans laquelle les séquences télomères en position 3' par rapport à la seconde séquence de marquage d'ADN intégrée à une positon sous-télomère comprennent un troisième site unique de reconnaissance par endonucléase et la méthode comprend en outre, avant l'étape (b), une étape d'introduction dans la cellule d'une troisième endonucléase qui clive le troisième site unique de reconnaissance par endonucléase dans lesdites séquences télomères.

16. Méthode suivant l'une quelconque des revendications 10 à 15, dans laquelle les taux du produit de gène sont modifiés en introduisant dans ladite cellule un acide nucléique qui est capable de diriger l'expression du produit de gène dans ladite cellule et en mettant en incubation la cellule dans des conditions qui provoquent l'expression de produit de gène.

17. Utilisation d'un produit d'assemblage d'acide nucléique dans une méthode pour analyser l'activité d'ALT dans des cellules eucaryotiques, dans laquelle le produit d'assemblage d'acide nucléique est apte à l'intégration d'une séquence de marquage d'ADN dans un télomère par recombinaison homologue, le produit d'assemblage comprenant la séquence de marquage d'ADN liée à (i) une première séquence d'ADN en position 3' de la séquence de marquage et (ii) une seconde séquence d'ADN en position 5' de la première séquence de marquage d'ADN, lesdites première et seconde séquences d'ADN comprenant chacune des séquences répétées multiples homologues de l'ADN télomère eucaryotique, et la séquence de marquage d'ADN comprenant un premier marqueur.

18. Utilisation suivant la revendication 17, dans laquelle les séquences répétées multiples sont des séquences (TTAGGG)n.

19. Utilisation suivant la revendication 17 ou la revendication 18, dans laquelle la séquence de marquage d'ADN comprend en outre un premier marqueur sélectionnable procaryotique en position 5' par rapport au premier marqueur, et le produit d'assemblage comprend un premier site de reconnaissance par endonucléase en position 3' du premier marqueur sélectionnable procaryotique.

20. Utilisation d'un produit d'assemblage d'acide nucléique dans une méthode d'analyse de l'activité d'ALT dans des cellules eucaryotiques, dans laquelle le produit d'assemblage d'acide nucléique est apte à l'intégration d'une séquence de marquage d'ADN à une position sous-télomère dans un chromosome, le produit d'assemblage comprenant la séquence de marquage d'ADN liée à (i) une première séquence d'ADN en position 3' de la séquence de marquage d'ADN, première séquence d'ADN qui comprend des séquences répétées multiples homologues de l'ADN télomère eucaryotique, et facultativement (ii) une troisième séquence d'ADN en position 5' de la séquence de marquage d'ADN, où n'importe quelle séquence d'ADN en position 5' de la séquence de marquage d'ADN ne contient pas de séquence d'acide nucléique homologue de l'ADN télomère eucaryotique, et la séquence de marquage d'ADN comprend un second marqueur.

21. Utilisation suivant la revendication 20, dans laquelle les séquences répétées multiples sont des séquences (TTAGGG)n.

22. Utilisation suivant la revendication 20 ou la revendication 21, dans laquelle la séquence de marquage d'ADN comprend en outre une origine bactérienne de réplication, un second marqueur sélectionnable procaryotique en position 3' par rapport au second marqueur et le produit d'assemblage comprend un second site de reconnaissance par endonucléase en position 5' du second marqueur sélectionnable procaryotique.
